(19) Europäisches Patentamt / European Patent Office / Office européen des brevets

(11) EP 1 875 239 B1

(12) **EUROPEAN PATENT SPECIFICATION**

(45) Date of publication and mention of the grant of the patent:
**05.10.2011 Bulletin 2011/40**

(21) Application number: **06751892.8**

(22) Date of filing: **27.04.2006**

(51) Int Cl.:
***G01N 33/534*** *(2006.01)* ***G01N 33/532*** *(2006.01)*

(86) International application number:
**PCT/US2006/016428**

(87) International publication number:
**WO 2006/116736 (02.11.2006 Gazette 2006/44)**

(54) **A METHOD FOR THE PREPARATION OF IMAGING PROBES USING CLICK CHEMISTRY**

VERFAHREN ZUR HERSTELLUNG VON ABBILDUNGSSONDEN UNTER VERWENDUNG VON CLICK CHEMIE

PROCEDE CHIMIQUE IN SITU DE CRIBLAGE DE SONDES D'IMAGERIE MOLECULAIRE DE HAUTE AFFINITE

(84) Designated Contracting States:
**AT BE BG CH CY CZ DE DK EE ES FI FR GB GR HU IE IS IT LI LT LU LV MC NL PL PT RO SE SI SK TR**

(30) Priority: **27.04.2005 US 675290 P**

(43) Date of publication of application:
**09.01.2008 Bulletin 2008/02**

(73) Proprietor: **Siemens Medical Solutions USA, Inc.**
**Malvern, PA 19355-1406 (US)**

(72) Inventors:
- **KOLB, Hartmuth, C.**
  **Playa del Rey, California 90293 (US)**
- **MOCHARLA, Vani, P.**
  **Los Angeles, California 90034 (US)**
- **WALSH, Joseph, C.**
  **Pacific Palisades, California 90272 (US)**

(74) Representative: **Hazzard, Alan David**
**Siemens Aktiengesellschaft**
**Postfach 22 16 34**
**80506 Munich (DE)**

(56) References cited:
**EP-A- 0 167 103** **WO-A-03/101972**
**WO-A-2004/057340**

- **KRASINSKI ANTONI ET AL: "In situ selection of lead compounds by click chemistry: Target-guided optimization of acetylcholinesterase inhibitors" JOURNAL OF THE AMERICAN CHEMICAL SOCIETY, vol. 127, no. 18, May 2005 (2005-05), pages 6686-6692, XP009072494 ISSN: 0002-7863**
- **MANETSCH ROMAN ET AL: "In situ click chemistry: Enzyme inhibitors made to their own specifications" JOURNAL OF THE AMERICAN CHEMICAL SOCIETY, vol. 126, no. 40, 13 October 2004 (2004-10-13), pages 12809-12818, XP009072495 ISSN: 0002-7863**
- **LEWIS W G ET AL: "Click chemistry in situ: acetylcholinesterase as a reaction vessel for the selective assembly of a femtomolar inhibitor from an array of building blocks" ANGEWANDTE CHEMIE. INTERNATIONAL EDITION, WILEY VCH VERLAG, WEINHEIM, DE, vol. 41, no. 6, 2002, pages 1053-1057, XP002967538 ISSN: 1433-7851**
- **NGUYEN R ET AL: "Using an Enzyme's Active Site To Template Inhibitors" ANGEWANDTE CHEMIE, WILEY-VCH, WEINHEIM, DE, vol. 40, no. 9, 2001, pages 1774-1776, XP002275869 ISSN: 1433-7851**
- **REES DAVID C ET AL: "Fragment-based lead discovery." NATURE REVIEWS. DRUG DISCOVERY. AUG 2004, vol. 3, no. 8, August 2004 (2004-08), pages 660-672, XP009072515 ISSN: 1474-1776**
- **KILBOURN M R ET AL: "SYNTHESIS OF FLUORINE-18 GBR-13119 A PRESYNAPTIC DOPAMINE UPTAKE ANTAGONIST" APPLIED RADIATION AND ISOTOPES, vol. 39, no. 4, 1988, pages 279-282, XP009072976 ISSN: 0883-2889**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 875 239 B1

**Description**

FIELD OF THE INVENTION

The invention relates to the use of *in situ* click chemistry methods for screening high affinity molecular imaging probes, such as PET probes.

BACKGROUND OF THE INVENTION

Positron Emission Tomography (PET) is a molecular imaging technology that is increasingly used for detection of disease. PET imaging systems create images based on the distribution of positron-emitting isotopes in the tissue of a patient. The isotopes are typically administered to a patient by injection of probe molecules that comprise a positron-emitting isotope, such as F-18, C-11, N-13, or O-15, covalently attached to a molecule that is readily metabolized or localized in the body (e.g., glucose) or that chemically binds to receptor sites within the body. In some cases, the isotope is administered to the patient as an ionic solution or by inhalation. One of the most widely used positron-emitter labeled PET molecular imaging probes is 2-deoxy-2-[$^{18}$F]fluoro-D-glucose ([$^{18}$F]FDG).

PET scanning using the glucose analog [$^{18}$F]FDG, which primarily targets glucose transporters, is an accurate clinical tool for the early detection, staging, and restaging of cancer. PET-FDG imaging is increasingly used to monitor cancer chemo- and chemoradiotherapy because early changes in glucose utilization have been shown to correlate with outcome predictions. A characteristic feature of tumor cells is their accelerated glycolysis rate, which results from the high metabolic demands of rapidly proliferating tumor tissue. Like glucose, FDG is taken up by cancer cells via glucose transporters and is phosphorylated by hexokinase to FDG-6 phosphate. The latter cannot proceed any further in the glycolysis chain, or leave the cell due to its charge, allowing cells with high glycolysis rates to be detected.

Although useful in many contexts, limitations of FDG-PET imaging for monitoring cancer exist as well. Accumulation in inflammatory tissue limits the specificity of FDG-PET. Conversely, nonspecific FDG uptake may also limit the sensitivity of PET for tumor response prediction. Therapy induced cellular stress reactions have been shown to cause a temporary increase in FDG-uptake in tumor cell lines treated by radiotherapy and chemotherapeutic drugs. Further, physiological high normal background activity (i.e., in the brain) can render the quantification of cancer-related FDG-uptake impossible in some areas of the body.

Due to these limitations, other PET imaging tracers are being developed to target other enzyme-mediated transformations in cancer tissue, such as 6-[F-18]fluoro-L-DOPA for dopamine synthesis, 3'-[F-18]Fluoro-3'-deoxythymidine (FLT) for DNA replication, and [C-11](methyl)choline for choline kinase, as well as ultra high-specific activity receptor-ligand binding (e.g., 16$\alpha$ [F-18]fluoroestradial) and potentially gene expression (e.g., [F-18]fluoro-ganciclovir). Molecularly targeted agents have demonstrated great potential value for non-invasive PET imaging in cancers.

These studies have demonstrated the great value of non-invasive PET imaging for specific metabolic targets of cancer. Despite the clear clinical value of incorporating PET imaging into patient management, limitations do exist. In certain instances, current imaging probes lack specificity or have inadequate signal to background characteristics. In addition, new biological targets that are being tested for therapeutic intervention will require new imaging probes to evaluate therapeutic potential.

Additional biomarkers are needed that show a very high affinity to, and specificity for, tumor targets to support cancer drug development and to provide health care providers with a means to accurately diagnose disease and monitor treatment. Such imaging probes could dramatically improve the apparent spatial resolution of the PET scanner, allowing smaller tumors to be detected, and nanomole quantities to be injected in patients.

Krasinsky et al. (2005) JACS 127, 6686-6692 discloses a process for the identification of an inhibitor for acetylcholine-esterase (AChE), comprising reacting AChE with a first ligand (TZ2), contacting the complex with a library for the in-situ selection of a second ligand, and coupling the first and second ligands using click chemistry. Manetsch et al. (2004) JACS 126, 12809-12818, describes an in-situ method for the identification of enzyme inhibitors comprising contacting AChE with a first library of ligands (TZ2-6, TA1-3), contacting the complex with a second library of ligands (PA2-5, PZ5-8), allowing a click reaction between members of the first and second library to take place, and identifying the formed products. EP 0167103 A discloses the preparation of compounds comprising $^{18}$F for use in PET, comprising transforming a compound of formula X-R to $^{18}$F-R, wherein X is a nucleophilic leaving group, such as a halo or nitro group.

SUMMARY OF THE INVENTION

The present invention provides a technology platform based on an *in situ* click chemistry approach (Mocharla, V. P.; Colasson, B.; Lee, L. V.; Roeper, S.; Sharpless, K. B.; Wong, C.-H.; Kolb, H. C. Angew. Chem. Int. Ed. 2005, 44, 116-120) to identify high-affinity PET probes that target biological macromolecules related to cancer and other diseases. High affinity ligands for biological targets are made through *in situ* click chemistry, by which the biological target templates

the assembly of two reactive fragments within the confines of its binding pockets. Radiolabeling of the target-generated ligands provides candidate PET imaging probes that may allow diagnosis and identification of tumor location, and may provide mechanistic information on tumor type for treatment. At least one of the paired fragments used in the screening process carries a non-radioactive isotope of an element (or chemical element) that includes a radioactive isotope within its nuclide that is suitable for use in molecular imaging probes (e.g., F-19) as part of its design in order to facilitate later introduction of the radionuclide (e.g., F-18).

In one embodiment, the present invention identifies a new class of molecular imaging probes for Carbonic anhydrase-II (CA-II). Physiologically, CA-II is one of 14 known isozymes of the carbonic anhydrase family and is expressed in almost every organ and tissue in the body. This is clearly a reflection on the importance of its ability to catalyze the reversible hydration of carbon dioxide into bicarbonate ion. This critical biological function makes CA-II a key player in processes that involve the transportation of $HCO_3^-/CO_2$ between tissues, pH control, bone resorption and electrolyte secretion in various epithelia. There are other members of the CA family, specifically isozymes CA-IX and CA-XII, which are reported to be overexpressed in cancer cells. By further applying this screening technology towards the identification of new CA-IX and CA-XII imaging agents, one may successfully image tumors that are CA-IX and CA-XII expressing and may ultimately lead towards the identification of novel therapeutics and therapy regimens.

In another embodiment, the screening platform was also applied towards the identification of novel, triazole-bearing cyclooxygenase-2 (COX-2) radioligands which may be use for imaging COX-2 expression in vivo. COX-2, an inducible member of the COX family that catalyzes the production of prostoglandins from arachadonic acid, is highly expressed in inflamed tissues. Upon the discovery of COX-2, a new class of COX-2 specific nonsteroidal anti-inflammatory drugs (NSAIDS) provided therapy for COX-2 mediated inflammatory-related diseases, the most common being rheumatoid arthritis. Because these COX-2 specific inhibitors selectively target COX-2 and not COX-1, the common side effects associated with traditional NSAID-related therapy, such as gastric bleeding, is not present. Though the new COX-2 therapy is not associated with COX-1 inhibiting side effects, COX-2 based therapeutics have received much attention as a result of purported cardiovascular problems associated with the therapeutic use of Rofecoxib (Vioxx®). From an in vivo imaging standpoint, monitoring the pharmacodynamics of compounds with unexplained side effects, such as COX-2 inhibitors, may provide insight and direction towards the development of safer therapeutics.

In another embodiment, the screening method is used to identify molecular imaging probes derived from an variety of biomacromolecules such as enzymes, receptors, DNA, RNA and antibodies. When these biomacromolecules are overexpressed or over-activated in vivo, they are detected through their binding to the molecular imaging probe. For example, in vivo imaging of tissues expressing high levels of carbonic anhydrase-II (CA-II) (e.g. red blood cells) may be achieved by administering a high affinity CA-II radioligand, identified by this screening process. In another embodiment, the screening technology can also be applied for identifying tumors that highly express signature biomacromolecules. For example, in the area of oncology imaging, radioligands that specifically target oncogenic proteins of the kinase family can lead to detection of a specific tumor phenotype. Particularly preferred target kinases belong to the PI-3-Kinase/AKT signaling pathway (PI-3-kinase: Phosphoinositol 3-kinase; Akt: Protein Kinase B), which relates to cell survival in cancer. For example, the kinase Akt is a key node in the oncogenic transformation of cells, making it a key target for developing imaging probes that penetrate cells and seek out activated Akt with high specificity and affinity.

In one embodiment, the screening method involves identifying a plurality of molecules that may exhibit affinity for the binding site of the enzyme and covalently attaching a non-radioactive isotope of an element (e.g., F-19) that includes at least one radioactive isotope in its nuclide (e.g., F-18) to the molecule. A functional group capable of participating in a click chemistry reaction, such as an azide or alkynyl group, is also attached to the molecule, optionally via a linker or linkers. Individual members of the resulting plurality of molecules are then mixed with the target molecule and individual members of a plurality or library of compounds that may exhibit affinity for a substrate binding site of the enzyme. The members of the substrate-binding library have been chemically modified to include a click chemistry functional group compatible with the functional group of the library of complimentary click fragment molecules. In one particular embodiment, any pair of compounds, one from each library, that exhibits affinity for the two targeted binding sites of the target kinase will covalently bond via the click chemistry functional groups *in situ.* These ligands are identified by known methods such as selected ion monitoring mass spectrometry (SIM/MS). Following chemical synthesis of these compounds, they are assayed, using conventional bioassay techniques, to determine their binding constants. Those ligands with sufficiently high binding affinities (with $IC_{50}$ values typically below 100 nM and above 1 fM), are considered hit compounds and are candidates for becoming molecular imaging probes. However, if the initial screen does not reveal compounds with sufficient binding affinities, a new screen is started and the iterative process repeats until an optimal candidate imaging probe is identified. The hit compounds are then converted into a molecular imaging probe via standard radiochemical techniques.

## BRIEF DESCRIPTION OF THE DRAWINGS

Having thus described the invention in general terms, reference will now be made to the accompanying drawings,

which are not necessarily drawn to scale, and wherein:

Fig. 1 is a schematic representation of an *in situ* click chemistry process;

Fig. 2 is a schematic representation of an embodiment of the *in situ* click chemistry method of the invention for screening ligands using a kinase template;

Fig. 3a illustrates two paths along which ligands may be formed inside Akt; and

Fig. 3b is an X-ray crystal structure of Akt revealing a close distance between the ribose moiety and the arginine residue (path A) and a close distance between the phosphate group of ATP and the serine residue (path B).

DETAILED DESCRIPTION OF THE PREFERRED EMBODIMENTS

The present invention now will be described more fully hereinafter. This invention may, however, be embodied in many different forms and should not be construed as limited to the embodiments set forth herein; rather, these embodiments are provided so that this disclosure will be thorough and complete, and will fully convey the scope of the invention to those skilled in the art.

## I. Definitions

As used herein, the singular forms "a", "an", "the", include plural referents unless the context clearly dictates otherwise.

"Alkyl" refers to a hydrocarbon chain, typically ranging from about 1 to 20 atoms in length. Such hydrocarbon chains may be branched or straight chain, although typically straight chain is preferred. Exemplary alkyl groups include ethyl, propyl, butyl, pentyl, 1-methylbutyl, 1-ethylpropyl, 3-methylpentyl, and the like. As used herein, "alkyl" includes cycloalkyl when three or more carbon atoms are referenced.

"Anchor site" as used herein is synonymous with the first binding site.

"Aryl" means one or more aromatic rings, each of 5 or 6 core carbon atoms. Aryl includes multiple aryl rings that may be fused, as in naphthyl or unfused, as in biphenyl. Aryl rings may also be fused or unfused with one or more cyclic hydrocarbon, heteroaryl, or heterocyclic rings. As used herein, "aryl" includes heteroaryl.

A "biological target" can be any biological molecule involved in biological pathways associated with any of various diseases and conditions, including cancer (e.g., leukemia, lymphomas, brain tumors, breast cancer, lung cancer, prostate cancer, gastric cancer, as well as skin cancer, bladder cancer, bone cancer, cervical cancer, colon cancer, esophageal cancer, eye cancer, gallbladder cancer, liver cancer, kidney cancer, laryngeal cancer, oral cancer, ovarian cancer, pancreatic cancer, penile cancer, glandular tumors, rectal cancer, small intestine cancer, sarcoma, testicular cancer, urethral cancer, uterine cancer, and vaginal cancer), diabetes, neurodegenerative diseases, cardiovascular diseases, respiratory diseases, digestive system diseases, infectious diseases, inflammatory diseases, autoimmune diseases, and the like. Exemplary biological pathways include, for example, cell cycle regulation (e.g., cellular proliferation and apoptosis), angiogenesis, signaling pathways, tumor suppressor pathways, inflammation (COX-2), oncogenes, and growth factor receptors. The biological target may also be referred to as the "target biomacromolecule" or the "biomacromolecule." The biological target can be a receptor, such as enzyme receptors, ligand-gated ion channels, G-protein-coupled receptors, and transcription factors. The biologically target is preferably a protein or protein complex, such as enzymes, membrane transport proteins, hormones, and antibodies. In one particularly preferred embodiment, the protein biological target is an enzyme, such as carbonic anhydrase-II and its related isozymes such as carbonic anhydrase IX and XII.

"Complementary functional groups" as used herein, means chemically reactive groups that react with one another with high specificity (i.e., the groups are selective for one another and their reaction provides well-defined products in a predictable fashion) to form new covalent bonds.

"Cycloalkyl" refers to a saturated or unsaturated cyclic hydrocarbon chain, including bridged, fused, or spiro cyclic compounds, preferably made up of 3 to about 12 carbon atoms, more preferably 3 to about 8.

"Heteroaryl" is an aryl group containing from one to four heteroatoms, preferably N, O, or S, or a combination thereof. Heteroaryl rings may also be fused with one or more cyclic hydrocarbon, heterocyclic, aryl, or heteroaryl rings.

"Heterocycle" or "heterocyclic" means one or more rings of 5-12 atoms, preferably 5-7 atoms, with or without unsaturation or aromatic character and having at least one ring atom which is not a carbon. Preferred heteroatoms include sulfur, oxygen, and nitrogen.

A "kinase" as used herein and also defined as well known in the art, is an enzyme that transfers a phosphate from adenosine triphosphate (ATP) onto a substrate molecule. A kinase includes a binding site for ATP, which is a

cofactor in the phosphorylation, and at least one binding site for the substrate molecule, which is typically another protein.

"Leaving group", as used herein refers to groups that are readily displaced, for example, by a nucleophile, such as an amine, a thiol or an alcohol nucleophile or its salt. Such leaving groups are well known and include, for example carboxylates, N-hydroxysuccinimide, N-hydroxybenzotriazole, halides, triflates, tosylates, -OR and -SR and the like.

A "ligand" is a molecule, preferably having a molecular weight of less than about 800 Da., more preferably less than about 600 Da., comprising a first group exhibiting affinity for a first binding site on a biological target molecule, such as a protein, and a second group exhibiting affinity for a second binding site on the same biological target molecule. The two binding sites can be separate areas within the same binding pocket on the target molecule. The ligands preferably exhibit nanomolar binding affinity for the biological target molecule. In certain aspects as disclosed herein, a ligand is used synonoously with a "substrate." A ligand may comprise a "molecular structure" as defined herein.

A "linker" as used herein refers to a chain comprising 1 to 10 atoms and may comprise of the atoms or groups, such as C, -NR-, O, S, -S(O)-, $-S(O)_2-$, CO, -C(NR)-and the like, and wherein R is H or is selected from the group consisting of $(C_{1}$-$_{10})$alkyl, $(C_{3-8})$cycloalkyl, aryl$(C_{1-5})$alkyl, heteroaryl$(C_{1-5})$alkyl, amino, aryl, heteroaryl, hydroxy, $(C_{1-10})$ alkoxy, aryloxy, heteroaryloxy, each substituted or unsubstituted. The linker chain may also comprise part of a saturated, unsaturated or aromatic ring, including polycyclic and heteroaromatic rings.

A "metal chelating group" as used herein, is as defined in the art, and may include, for example, a molecule, fragment or functional group that selectively attaches or binds metal ions, and forms a complex. Certain organic compounds may form coordinate bonds with metals through two or more atoms of the organic compound. Examples of such molecule include DOTA, EDTA, and porphine.

"Molecular structure" refers to a molecule or a portion or fragment of a molecule that is attached to the click functional group, optionally attached to a leaving group and/or radioactive isotope or, in certain variations, the molecule may be attached to a linker that is attached to the click functional group. Non-exclusive examples of such molecular structures include, for example, a substituted or unsubstituted methylene, alkyl groups (C1-C10) that are linear or branched, each optionally comprising a heteroatoms selected from the group consisting of O, N and S, aryl and heteroaryl groups each unsubstituted or substituted, biomacromolecules, nucleosides and their analogs or derivatives, peptides and peptide mimics, carbohydrates and combinations thereof.

"Polydentate metal chelating group" means a chemical group with two or more donator atoms that can coordinate to (i.e. chelate) a metal simultaneously. Accordingly, a polydentate group has two or more donor atoms and occupies two or more sites in a coordination sphere.

The terms "patient" and "subject" refer to any human or animal subject, particularly including all mammals.

The term "pericyclic reaction" refers to a reaction in which bonds are made or broken in a concerted cyclic transition state. A concerted reaction is one which involves no intermediates during the course of the reaction. Typically, there is a relatively small solvent effect on the rate of reaction, unless the reactants themselves happen to be charged, i.e. carbonium or carbanions.

As used herein, "radiochemical" is intended to encompass any organic, inorganic or organometallic compound comprising a covalently-attached radioactive isotope, any inorganic radioactive ionic solution (e.g., $Na[^{18}F]F$ ionic solution), or any radioactive gas (e.g., $[^{11}C]CO_2$), particularly including radioactive molecular imaging probes intended for administration to a patient (e.g., by inhalation, ingestion, or intravenous injection) for tissue imaging purposes, which are also referred to in the art as radiopharmaceuticals, radiotracers, or radioligands. Although the present invention is primarily directed to synthesis of positron-emitting molecular imaging probes for use in PET imaging systems, the invention could be readily adapted for synthesis of any radioactive compound comprising a radionuclide, including radiochemicals useful in other imaging systems, such as single photon emission computed tomography (SPECT).

As used herein, the term "radioactive isotope" refers to isotopes exhibiting radioactive decay (i.e., emitting positrons) and radiolabeling agents comprising a radioactive isotope (e.g., $[^{11}C]$methane, $[^{11}C]$carbon monoxide, $[^{11}C]$ carbon dioxide, $[^{11}C]$phosgene, $[^{11}C]$urea, $[^{11}C]$cyanogen bromide, as well as various acid chlorides, carboxylic acids, alcohols, aldehydes, and ketones containing carbon-11). Such isotopes are also referred to in the art as radioisotopes or radionuclides. Radioactive isotopes are named herein using various commonly used combinations of the name or symbol of the element and its mass number (e.g., $^{18}F$, F-18, or fluorine-18). Exemplary radioactive isotopes include I-124, F-18 fluoride, C-11, N-13, and O-15, which have half-lives of 4.2 days, 110 minutes, 20 minutes, 10 minutes, and 2 minutes, respectively. The radioactive isotope is preferably dissolved in an organic solvent, such as a polar aprotic solvent. The radioactive isotope used in the present method is F-18.

Optical imaging agent refers to molecules that have wavelength emission greater than 400nm and below 1200nm. Examples of optical imaging agents are Alex Fluor, BODIPY, Nile Blue, COB, rhodamine, Oregon green, fluorescein and acridine.

The term "reactive precursor" is directed to any of a variety of molecules that can be chemically modified by addition of an azide or alkynyl group, such as small molecules, natural products, or biomolecules (e.g., peptides or proteins). For ligand formation from two precursor molecules, one of the precursor molecules comprises a non-radioactive isotope of an element having a radioisotope within its nuclide. In certain aspects as used herein, the term "ligand" may

refer to the precursor, compounds and imaging probes that bind to the biomacromolecule. The two precursors of the ligand preferably exhibit affinity to separate binding sites (or separate sections of the same binding site or pocket) on a biological target molecule, such as an enzyme. The reactive precursor that has binding affinity for an active site on the biomacromolecule is sometimes referred to herein as the "anchor molecule." The reactive precursor that has binding affinity for the substrate binding site of a kinase is sometimes referred to herein as the "substrate mimic." The term "reactive precursor" may also refer to the precursor or compound that are used to prepare the candidate compounds that comprise the library of candidate compounds.

In a particular aspect of the method with the ligand radiochemical embodiment, one of the precursor molecules may also comprise a leaving group that can be readily displaced by nucleophilic substitution in order to covalently attach a radioisotope to the precursor. Exemplary reactive precursors include small molecules bearing structural similarities to existing PET probe molecules, EGF, cancer markers (e.g., p185HER2 for breast cancer, CEA for ovarian, lung, breast, pancreas, and gastrointestinal tract cancers, and PSCA for prostrate cancer), growth factor receptors (e.g., EGFR and VEGFR), glycoproteins related to autoimmune diseases (e.g., HC gp-39), tumor or inflammation specific glycoprotein receptors (e.g., selectins), integrin specific antibody, virus-related antigens (e.g., HSV glycoprotein D, EV gp), and organ specific gene products.

"Substituted" or a "substituent" as used herein, means that a compound or functional group comprising one or more hydrogen atom of which is substituted by a group (a substituent) such as a $-C_{1-5}$alkyl, $C_{2-5}$alkenyl, halogen (chlorine, fluorine, bromine, iodine atom), $-CF_3$, nitro, amino, oxo, -OH, carboxyl, $-COOC_{1-5}$alkyl, $-OC_{1-5}$alkyl, $-CONHC_{1-5}$alkyl, $-NHCOC_{1-5}$alkyl, $-OSOC_{1-5}$alkyl, $-SOOC_{1-5}$alkyl, $-SOONHC_{1-5}$alkyl, $-NHSO_2C_{1-5}$alkyl, aryl, heteroaryl and the like, each of which may be further substituted.

"Substrate mimics" as used herein means compounds that imitate enzyme substrates in their 3-dimensional structures, charge distribution and hydrogen bond donor or acceptor orientation, so they can be recognized by the enzyme active site.

## II. ***In Situ*** Click Chemistry Method

The traditional method for the preparation of molecular imaging probes begins with the identification of a tightly binding molecule. This molecule may have been previously identified from a large screen or by SAR development. The compound is then assessed in terms of likelihood of radiolabeling as well as for preferential labeling sites on the molecule itself. Once the position for radiolabeling is determined, the compound is converted into an imaging agent by labeling with 18F-fluoride if used for PET imaging, injected into an appropriate living organism such as a mouse and then imaged with a PET scanner to determine the tracer's biodistribution, pharmacokinetic and pharmacodynamic profiles, metabolism and excretion pathways. In the instances where the radioactive element introduced into the molecule was not present in the original molecule as its non-radioactive isotope, there exists as possibility that the labeled compound will behave very differently than the parent compound. For example, introduction of 18F-fluoride in place of a hydrogen atom can cause a weakening of the imaging probe's binding affinity towards the target. This weakening may prevent the tracer from localizing in vivo thus preventing the formation of an adequate PET image.

An alternative approach towards identifying molecular imaging probes would be to prepare a library of non-radioisotope containing ligands and screen each individual ligand for potential activity in vitro. Once a hit is found, this molecule is converted into its radioactive analog and imaged in vivo. This radioactive analog of the parent compound is expected to possess the same physiochemical characteristics as the parent compound because of the similarity in sterics and electronics of the radioisotope. The drawback to this method is that a potentially large number of compounds must be prepared, identified, purified and individually screened for activity. This process requires much time, capital investment and effort. A third method, which uses the biological target as the reaction vessel to assemble its preferred potential molecular imaging probe through the use of click chemistry, would exhibit distinct advantages over the previous two methods.

Click chemistry provides an opportunity to design small molecule PET imaging tracers, which display extremely high affinities for their biological targets, setting the stage for "high-performance" molecular imaging with excellent signal to background ratios. Click chemistry is a modular approach to chemical synthesis that utilizes only the most practical and reliable chemical transformations. This technique produces high-affinity inhibitors by assembling building block reagents irreversibly inside a target's binding pockets. The general approach of *in situ* click chemistry is presented in Figure 1. Click chemistry techniques are described, for example, in the following references, which are incorporated herein by reference in their entirety:

- Kolb, H. C.; Finn, M. G.; Sharpless, K. B. Angewandte Chemie, International Edition 2001, 40, 2004-2021.
- Kolb, H. C.; Sharpless, K. B. Drug Discovery Today 2003, 8, 1128-1137.
- Rostovtsev, V. V.; Green, L. G.; Fokin, V. V.; Sharpless, K. B. Angewandte Chemie, International Edition 2002, 41, 2596-2599.

- Tornøe, C. W.; Christensen, C.; Meldal, M. Journal of Organic Chemistry 2002, 67, 3057-3064.
- Wang, Q.; Chan, T. R.; Hilgraf, R.; Fokin, V. V.; Sharpless, K. B.; Finn, M. G. Journal of the American Chemical Society 2003, 125, 3192-3193.
- Lee, L. V.; Mitchell, M. L.; Huang, S.-J.; Fokin, V. V.; Sharpless, K. B.; Wong, C.-H. Journal of the American Chemical Society 2003, 125, 9588-9589.
- Lewis, W. G.; Green, L. G.; Grynszpan, F.; Radic, Z.; Carlier, P. R.; Taylor, P.; Finn, M. G.; Barry, K. Angew. Chem., Int. Ed. 2002, 41, 1053-1057.
- Manetsch, R.; Krasinski, A.; Radic, Z.; Raushel, J.; Taylor, P.; Sharpless, K. B.; Kolb, H. C. Journal of the American Chemical Society 2004, 126, 12809-12818.
- Mocharla, V. P.; Colasson, B.; Lee, L. V.; Roeper, S.; Sharpless, K. B.; Wong, C.-H.; Kolb, H. C. Angew. Chem. Int. Ed. 2005, 44, 116-120.
- M. Whiting, J. Muldoon, Y.-C. Lin, S. M. Silverman, W. Lindstrom, A. J. Olson, H. C. Kolb, M. G. Finn, K. B. Sharpless, J. H. Elder, V. V. Fokin, Angew. Chem. 2006, 118, 1463-1467; Angew. Chem. Int. Ed. Engl. 2006, 45, 1435-1439.


Although other click chemistry functional groups can be utilized, such as those described in the above references, the use of cycloaddition reactions is preferred, particularly the reaction of azides with alkynyl groups. Alkynes, such as terminal alkynes and azides undergo 1,3-dipolar cycloaddition forming 1,4-disubstituted 1,2,3-triazoles. Alternatively, a 1,5-disubstituted 1,2,3-triazole can be formed using azide and alkynyl reagents (Krasinski, A., Fokin, V.V. & Barry, K. Organic Letters 2004, 1237-1240). Hetero-Diels-Alder reactions or 1,3-dipolar cycloaddition reactions could also be used (see Huisgen 1,3-Dipolar Cycloaddition Chemistry (Vol. 1) (Padwa, A., ed.), pp. 1-176, Wiley; Jorgensen Angew. Chem. Int. Ed. Engl. 2000, 39, 3558-3588; Tietze, L.F. and Kettschau, G. Top. Curr. Chem. 1997, 189, 1-120). These reactions are catalyzed by the biological target molecule *in situ* within its binding pockets. In contrast to the biological target catalyzed reactions, the thermal 1,3-dipolar cycloaddition reaction between azide and acetylene or alkynyl reactive precursors, which carry binding groups for either site, is extremely slow at room temperature, and the reactants are bio-orthogonal (i.e., they do not react with biological molecules and are largely inert under physiological conditions) thus supporting the claim that the inhibitory compounds are generated in situ. This minimization of the non-catalyzed reaction prevents the unwanted formation and identification of non-templated target ligands leading to false positives in the screening method.

In one particular embodiment, the in situ click chemistry method as discussed herein generates novel ligands with sub-nanomolar potencies that carry the PET label as part of the design. During the ligand discovery phase, a non-radioactive or 'cold' isotope (e.g., F-19) is used as a place-holder for the PET radionuclide. Once a high-affinity ligand is found, the F-19 is replaced by F-18. This target-guided strategy utilizes the enzyme itself as a template for generating potent ligand inhibitors from 'monovalent' building block reagents that are selectively bound to the enzyme and then irreversibly linked to each other within the confines of its binding pockets. Since this approach employs the biological target to assemble its own inhibitors from relatively few reagents (which may be combined in thousands of different ways), rather than requiring the synthesis, purification, and screening of thousands of library compounds, it promises to be more efficient than traditional combinatorial chemistry. Follow-up tests of the enzyme-generated hits then establish their binding affinity to and specificity for the target. In a particular embodiment, bivalent molecules that have multiple interactions with the biomacromolecule, such as a protein, the resulting hits are very potent. The resulting bivalent molecules bind to the co-factor binding site and reach into the substrate pocket. Mainly for entropy reasons (avoidance of the loss of three degrees of rotational and translational freedom), ligand inhibitors display much higher affinity to their biological targets than the individual components. Thus, even fragments with only modest micromolar affinity to individual binding pockets can generate nanomolar inhibitors when coupled together to permit optimal binding interactions with the biological target. Thus, the binding affinity of the building block reagent or precursor to the enzyme does not need to be in the nanomolar range. Micromolar affinity is sufficient for the click chemistry reaction to occur.

*In situ* click chemistry offers an attractive new approach to molecular probe discovery, since it is not dependent on the screening of final compounds, laboriously prepared through traditional means, but rather allows the enzyme to select and combine building blocks that fit into its binding site to assemble its own inhibitor molecules. For example, with just 200 building blocks (100 mono-azides and 100 mono-acetylenes), one can quickly scan through 20,000 possible combinations (100 x 100 x 2; the factor '2' accounts for possible *syn*- or *anti*-triazole formation) without actually having to make these compounds. This number becomes even larger, with the same number of building blocks, if one includes di- or tri-azides or -acetylenes, thereby providing the enzyme with greater flexibility to choose the appropriate building block and functional group at the same time. The screening method is as simple as determining whether or not the product has been formed in a given test mixture by LC/MS. A compound that is formed by the enzyme is likely to be a good and selective binder, due to the multivalent nature of the interaction.

Ligand development or the method for identifying imaging probes may occur in two stages. in certain embodiments, during the discovery phase, each compound carries a 'cold' F-19 atom, to prepare for later introduction of 'hot' F-18 without changing the compound's binding affinity to the enzyme appreciably. The precursors are compounds that

adhere to the target's binding pocket with low micromolar or better affinity, and that carry a bio-orthogonal functional group (azide or acetylene). In one embodiment of the method, each molecule carries one fluorine atom, which will later be replaced by the F-18 radionuclide. In certain embodiments, it is preferable to introduce the fluorine atom in the anchor molecule, rather than the substrate mimic or the linker module, since this will minimize the synthetic effort - fewer fluorine-containing compounds will need to be made, since the total number of potential anchor molecules is much smaller than that of the substrate/linker moieties.

In one embodiment, the screening method involves identifying a plurality of molecules that exhibit affinity for the binding site of the target enzyme and covalently attaching a non-radioactive isotope of an element that includes at least one radioactive isotope in its nuclide (e.g., F-19) to the molecule. A functional group capable of participating in a click chemistry reaction, such as an azide or alkynyl group, is also attached to the molecule, optionally via a linker. Individual members of the resulting plurality of molecules are then mixed with the target molecule and individual members of a plurality or library of compounds that may exhibit affinity for a substrate binding site of the enzyme. The members of the substrate-binding library have been chemically modified to include a click chemistry functional group compatible with the functional group of the library of cofactor-binding molecules. Thus, any pair of compounds, one from each library, that exhibits affinity for the binding sites of the enzyme will covalently bond via the click chemistry functional groups *in situ*. The screening process can utilize conventional screening equipment known in the art such as multi-well microtiter plates.

A mass spectrometer may be used for sequential, automated data analysis of the screening process. Exemplary spectrometer equipment that can be used include the Agilent MSD 1100 SL system, linear ion trap systems (ThermoFinnigan LTQ), quadrupole ion trap (LCQ), or a quadrupole time-of-flight (QTOF from Waters or Applied Biosystems). Each of these analyzers have very effective HPLC interfaces for LC-MS experiments.

In one embodiment, the starting precursor fragment, that may be an anchor molecule, discovery can be performed by designing small, targeted compound libraries (e.g., less than 100 compounds) based on known drugs and/or substrates. These libraries may be screened using traditional binding assays. The anchor molecules may be incubated with the enzyme target and small libraries of complementary click chemistry reagents or precursors (e.g., acetylenes, if the anchor molecule is an azide). Each reaction mixture may be analyzed by LC/MS to identify products that are formed by the enzyme. Hit validation is performed through competition experiments to demonstrate that the compound is indeed formed by the enzyme, and binding assays may establish the binding affinities of the enzyme-generated hits.

In one embodiment, the anchor molecules carry bio-orthogonal functional groups (e.g., $-N_3$, $-C\equiv CR$, where R=H, alkyl, aryl etc.) for their later conversion into ligand compounds inside the enzyme. Preferably, R is a hydrogen. In addition, each candidate anchor molecule carries one fluorine atom, which is easily introduced by nucleophilic substitution chemistry, to enable later [F-18] labeling by replacing 'cold' [F-19] with the corresponding radionuclide. This change is expected to have a minimal effect on the binding affinity, thereby making PET probe development more predictable.

The nature and the length of the linker between the two reacting groups or precursors may be selected to afford compounds with optimal binding affinities. Therefore, various types of linkers can be attached to the substrate mimics discussed above. This can readily be accomplished through carbon-heteroatom bond-forming reactions, which involve the azide groups either directly (triazole formation) or indirectly (azide reduction, followed by acylation or sulfonylation of the resulting amines). The library of substrate mimics preferably includes di-azides and di-acetylenes to increase the number of possible combination reactions.

**Aspects of The Invention:**

Aspects and embodiments of the invention are set out in the accompanying claims.

In one embodiment, there is provided a method for identifying a candidate imaging probe, the method comprising:

a) contacting a first library of candidate compounds with a target biomacromolecule, each compound of the first library of compounds comprises a first functional group capable of participating in a click chemistry reaction, and each compound optionally exhibiting affinity for a first binding site of the target biomacromolecule that comprises the first binding site and a second binding site;

b) identifying a first member from the first library of candidate compounds exhibiting affinity for the first binding site;

c) contacting the first member identified from the first library of candidate compounds exhibiting affinity for the first binding site with the target biomacromolecule;

d) contacting a second library of candidate compounds with the first member and the target biomacromolecule, the second library of candidate compounds optionally exhibiting affinity for the second binding site, wherein each compound of the second library of candidate compounds comprises a complementary second functional group capable of participating in a click chemistry reaction with the first functional group, wherein either each compound of the first library or each compound of the second library, or each compound of both the first and the second library of compounds, independently comprises a non-radioactive isotope of a chemical element and wherein the chemical element comprises at least one radioisotope, optionally attached via a linker, wherein the non-radioactive isotope is F-19, and wherein the radioactive isotope is F-18;

e) reacting the complementary second functional group with the first functional group via a biomacromolecule induced click chemistry reaction to form the candidate imaging probe;

f) isolating and identifying the candidate imaging probe;

g) preparing the candidate imaging probe by chemical synthesis; and

h) converting the candidate imaging probe into an imaging probe by converting the non-radioactive isotope of the element into the radioactive isotope F-18. In one variation of the method, the biomacromolecule is selected from the group consisting of enzymes, receptors, DNA, RNA, ion channels and antibodies. In another variation, the target biomacromolecule is a protein that is overexpressed in disease states, such as beta-amyloid in brain tissue of Alzheimer's Disease patients.

In another aspect of the above method, the second binding site constitutes a portion of the first binding site. In one variation of the method, each compound of the first library or each compound of the second library, or each compound of both the first and the second library of compounds comprises a metal chelating group, and/or a fluorophore. In a particular variation, the click chemistry reaction is a pericyclic reaction. In another variation of the above, the pericyclic reaction is a cycloaddition reaction. In yet another variation, the cycloaddition reaction is selected from the group consisting of a Diels-Alder reaction or a 1,3-dipolar cycloaddition reaction. Preferably, in certain methods used herein, the cycloaddition reaction is a 1,3-dipolar cycloaddition reaction.

In yet another aspect of the above method, the complementary click functional groups comprises an azide and an alkyne and the click reaction forms a 1,2,3 triazole comprising product. In one variation, the first functional group is an azide and the second functional group is an alkyne, or wherein the first functional group is an alkyne and the second functional group is an azide. In another variation, the alkyne is a terminal alkyne. In a particular variation of the above method, steps of a) to f) are performed in an iterative procedure of preparing a new first library of compounds and/or second library of compounds and re-screening until a candidate imaging probe with optimized binding, biodistribution, metabolism and pharmacokinetic properties is identified.

In a particular aspect, the identified imaging probe exhibits high binding affinity and specificity for the targeted biomacromolecule. Preferably, the binding affinity is of nanomolar or better, and the identified imaging probe exhibits optimal biodistribution, metabolism, pharmacokinetic and clearance properties.

In one variation of the above method, the leaving group may be converted to form a labeled derivative by an exchange reaction, a nucleophilic substitution reaction or by a electrophilic substitution reaction. In another variation of the above method, the identified candidate imaging probe is labeled with a radioactive isotope, and the resulting radioactive imaging probe is used for an imaging method selected from the group consisting of PET, SPECT and optical imaging.

In one aspect of the method, for certain biomacromolecule, the first binding site is a co-factor binding site, and the second binding site is a substrate binding site.

In one variation of the above methods, the target enzyme is selected from the group consisting of overexpressed or overactivated in disease states such as COX-2, AKT, P13K, or CA-9/CA-12. In another variation, the target biomacromolecule is a protein that is overexpressed in disease states, including beta-amyloid in brain tissue of Alzheimer's Disease patients. In yet another variation, the second binding site constitute a portion of the first binding site. In a particular aspect of the above method, the second binding site constitute a portion or a section of the first binding site and the binding of the second candidate compound binds to the binding site by capping the first binding site. In yet another variation of the above methods, each compound of the first library or each compound of the second library, or each compound of both the first and the second library of compounds comprises a metal chelating group, and/or a fluorophore. In another variation, the click chemistry reaction is a pericyclic reaction. Preferably, in certain variations, the pericyclic reaction is a cycloaddition reaction. In yet another variation, the cycloaddition reaction is selected from the group consisting of a Diels-Alder reaction or a 1,3-dipolar cycloaddition reaction. Preferably, in certain variations of the above, the cycloaddition reaction is a 1,3-dipolar cycloaddition reaction. In yet another variation, the complementary click functional groups comprises an azide and an alkyne and the click reaction forms a 1,2,3 triazole comprising product.

In a particular variation of the above method, the first functional group is an azide and the second functional

group is a terminal alkyne, or wherein the first functional group is a terminal alkyne and the second functional group is an azide. In another variation of the method, steps of a) to f) are performed in an iterative procedure of preparing a new first library of compounds and/or second library of compounds and re-screening until a candidate imaging probe having an optimized binding affinity is identified. In yet another variation, the leaving group is amenable to form a labeled derivative by an exchange reaction, a nucleophilic substitution reaction or by a electrophilic substitution reaction. In one aspect of the above method, the identified candidate imaging probe is labeled with a radioactive isotope, and the resulting radioactive imaging probe is used for an imaging method selected from the group consisting of PET, SPECT and optical imaging. In one variation of the above, the complementary click functional groups comprises an azide and an alkyne and the click reaction forms a 1,2,3 triazole comprising product. In yet another variation, the first functional group is an azide and the second functional group is an alkyne, or wherein the first functional group is an alkyne and the second functional group is an azide. In a particular aspect of the above, the alkyne employed in the click reaction is a terminal alkyne.

In a particular variation of the above method, steps of a) to f) are performed in an iterative procedure of preparing a new first library of compounds and/or second library of compounds and re-screening until a candidate imaging probe with optimized binding, biodistribution, metabolism and pharmacokinetic properties is identified.

In one particular aspect of the method, the method is performed with at least one iteration. In one variation, the method is performed with at least 5 iterations, at least 10 iterations or at least 20 iterations until a candidate imaging probe having an optimized binding, biodistribution, pharmacokinetics, metabolism and clearance properties is identified. In the method disclosed herein, the optimized binding affinity is defined as being in the nanomolar or better range. Optimized biodistribution and pharmacokinetics means that the compound reaches the targeted tissue in vivo and that it stays in the blood stream in a sufficient period of time, such as from several minutes to 2 hours in case of 18-F PET, for example, to allow the compound or imaging probe to be bound to the targeted biomolecule in vivo. Optimized metabolism means that the compound or probe doesn't get metabolized with formation of inactive products or, worse, radioactive compounds or ions that target other, undesired, tissues. As an example, if the compound loses its radioactive 18F, it will give a strong, undesired, PET signal in the bone. Optimized clearance properties means that the unbound compound or probe, which has not reached its target tissue, is rapidly cleared from the organism (within 2-3 hours at the latest, in case of 18F), to give a large signal to background ratio ("signal" means the signal, e.g. radioactivity, that emanates from the bound compound).

In another aspect of the present method, the first binding site is the substrate binding site and the second binding site is a cofactor binding site, or the first binding site is a cofactor binding site and the second binding site is the substrate binding site.

In a particular variation of the above method, the leaving group may be converted to form a labeled derivative by an exchange reaction, a nucleophilic substitution reaction, an electrophilic substitution reaction or by forming a complex with a radioactive metal. In a particular variation of the method, the leaving group is selected from the group consisting of halo, hydroxy, acyloxy, nitro and sulfonyloxy group. Specific examples of leaving group includes alkanoyloxy (e.g. acetoxy, propionyloxy, etc.), sulfonyloxy (e.g. mesyloxy, tosyloxy, etc ...), and the like.

In a particular variation of the method, the identified ligand compound is labeled with a radioactive isotope, and the resulting ligand compound is used for an imaging methods selected from the group consisting of PET, SPECT and optical imaging. For particular application of the above method, the radioactive isotope is F-18. In a variation of the method, the binding of the candidate compounds within the enzyme binding sites facilitates the click chemistry reaction in the absence of any externally added catalyst. In yet another variation of the method, the second library of candidate compounds comprises of 1 or more compounds. In a particular variation of the method, the first library of candidate compounds and the second library of candidate compounds each independently comprise at least one compound, at least five compounds, at least ten or more compounds, or at least 25 or more compounds.

In another aspect of the above method, the first library of candidate compounds and/or the second library of candidate compounds further comprises a linker between the compound and the first functional group and/or a linker between the compound and the second functional group. In one variation, the linker comprises between 1 to 10 atoms in the linker chain between the compound and the functional group.

In another embodiment, there is provided a method according to the above, wherein the first library of candidate compounds comprises compounds of the formula I, and the second library of candidate compounds comprises compounds of the formula II:

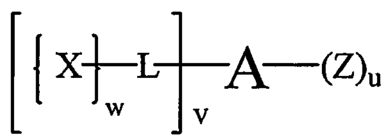

I

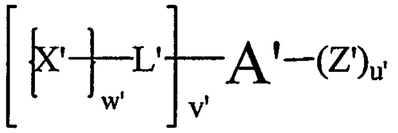

II

wherein: A and A' are each independently a substrate mimic selected from the group consisting of a substituted or unsubstituted aryl or heteroaryl group, non-peptide substrate mimic, peptidomimetic and arginine group mimics; L and L' are each independently a bond or a linking group comprising 1 to 10 atoms in the linking chain, optionally substituted by 0-3 substituents; X and X' are each independently a complementary click chemistry functional group; Z and Z' are each independently an $NH_2$, hydrazinyl, substituted hydrazinyl, and optionally substituted urea, or are absent; U and U' are each independently 0, 1, 2 or 3; V and V' are each independently 1, 2 or 3; and W and W' are each independently 1, 2 or 3.

In each of the above aspects of the disclosure as recited herein, including all aspects, embodiments and variations and representative examples, are intended to be interchangeable where applicable, such that the various aspects, embodiments and variations may be combined interchangeably and in different permutations. For example, a particular first molecular structure comprising a first functional group without a linker may undergo a 1,3-dipolar cycloaddition reaction with a second molecular structure with a complementary functional group without a linker, or alternatively, the same first molecular structure comprising the functional group with a linker may undergo a 1,3-dipolar cycloaddition reaction with a second molecular structure comprising a complementary functional group comprising a linker between the molecular structure and the complementary functional group. These and other permutations and variations are intended to be included in the aspects of the invention.

The reagent concentration is one important parameter, since it influences the rate of both the enzyme-induced reaction (i.e., the desired *in situ* reaction) as well as that of the enzyme-free background reaction. For practical reasons, it is desirable to keep the reagent concentrations low in order to minimize the extent of the background reaction, so that enzyme-based product formation can more easily be identified. At the same time, the concentration of the anchor molecule should be sufficiently high to accomplish significant saturation of the binding site for optimal use of the available enzyme. It is preferable to use anchor molecule and enzyme concentrations that are sufficiently high to achieve 95% or higher active site saturation. The last reaction component, the substrate mimic, should be available in sufficiently high concentrations to allow the bimolecular reaction between the enzyme/anchor molecule complex and the substrate mimic to take place with reasonable rates. Typically, the substrate mimic concentration will be at least 400 $\mu$M. The concentrations can be adjusted downwards or upwards, should the background reaction turn out to too fast or if very few *in situ* hits are found.

Each reagent/enzyme combination can be analyzed by LC-mass spectrometry to identify triazole products, which are potential in situ hits. Several tests can be performed to validate these potential in situ hits, by determining whether or not they were formed by the enzyme: (a) No-enzyme and BSA control experiments can identify false positives; (b) competitive inhibition of *in situ* product-formation in the presence of known enzyme inhibitors (e.g., ethoxzolamide in the case of CA-II) can reveal whether a given hit is formed in or near the active site of the enzyme. Validated *in situ* hits can be synthesized chemically and characterized in biological tests to determine their binding affinities. The substitution pattern of the triazole, since the enzyme may either form the 1,4-disubstituted or the 1,5-disubstituted isomer, can also be determined. This can be accomplished by making reference compounds using the $Cu^I$-catalyzed azide/acetylene reaction, which provides 1,4-disubstituted triazoles, the $Ru^{II}$-catalyzed azide/acetylene reaction, which provides 1,5-disubstituted triazoles or the thermal cycloaddition reaction, which produces mixtures of 1,4- and 1,5-disubstituted triazoles.

Many modifications and other embodiments of the invention will come to mind to one skilled in the art to which this invention pertains having the benefit of the teachings presented in the foregoing description. Therefore, it is to be understood that the invention is not to be limited to the specific embodiments disclosed and that modifications and other embodiments are intended to be included within the scope of the invention. Although specific terms are employed herein, they are used in a generic and descriptive sense only and not for purposes of limitation.

**Examples**

## CA-II IN SITU SCREENS

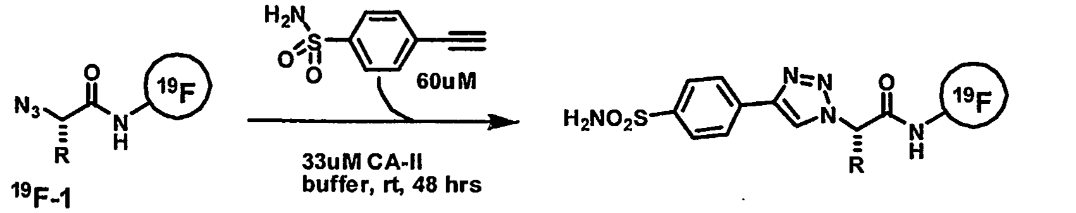


$^{19}$F-1

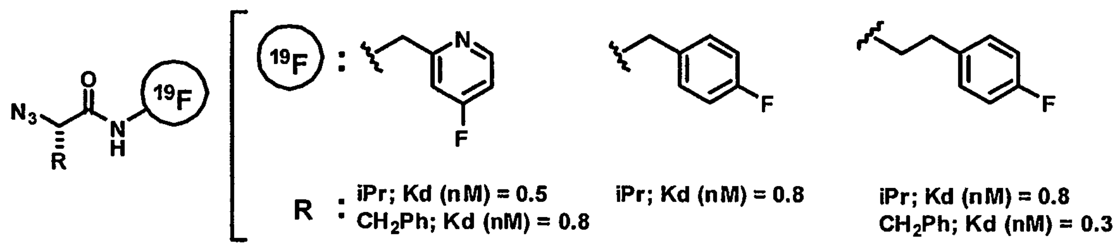


**General.** Carbonic anhydrase II from bovine erythrocytes (Sigma-Aldrich, catalog number C2522; lot number 083K9295, 4,014 Wilbur-Anderson units/mg, 90% protein content by Biuret) was used for *in situ* click chemistry experiments and for the determination of binding constants. We tested the protein by SDS gel electrophoresis and found it to display a single band corresponding to 29-30,000 molecular weight units. Carbonic anhydrase II from human erythrocytes (Sigma Aldrich, catalog number C-6165, 4,260 wilbur-anderson units/mg) was used for the determination of binding affinities for the human enzyme. All fluorescence measurements were performed on a SPECTRA MAX GEMINI fluorescence plate reader at 37 °C. The LC/MS analyses were performed on an Agilent 1100 series LC/MSD (SL) using a 30 X 2.1 mm Zorbax C8 column with a Phenomenex C18 pre-column. Compound detection was accomplished by electrospray mass spectroscopy in positive selected ion mode (LC/MS-SIM). The elution solvents,acetonitrile and water, contained 0.05% TFA.

**General procedure for *in situ* click chemistry experiments:**

Stock solutions of azides 19F-1 (20 mM), ethoxazolamide (20 mM) and ethynyl benzenesulfonamide (2 mM) were prepared in DMSO. The alkyne (3 μL) was added to wells of 96-well microtiter plates, containing the enzyme (94 μl of a 1 mg/mL solution, prepared from the commercial 90% pure protein by dissolution in pH 7.4 250mM potassium phosphate buffer), followed by the azide reagent (2 μL) and DMSO (1 μL). The reaction plate was stored at 37 °C for 40 hours.

The final reagent concentrations were as follows:

Enzyme (29 μM), alkyne (60 μM), azide (400 μM) and DMSO (6 vol-%). In parallel, several control reactions were set up and subjected to analogous experimental conditions: (1) Competitive inhibition control with ethoxazolamide (1 μL, 200 μM final concentration); (2) non-specific protein binding control using bovine serum albumin in place of bCA-II (1 mg/mL final concentration). In some cases, we also performed "no protein" control experiments, using pH 7.4 buffer instead of bCA-II. LC/MS-SIM analysis: All samples were analyzed by reverse phase HPLC with electrospray mass spectroscopic detection in the positive selected ion mode. The injection volume was 30 μL at a flow rate of 0.3 mL/min. The following elution gradient was employed: 0-100% acetonitrile/0.05% TFA and water/0.05% TFA over 16 minutes, 100% acetonitrile/0.05% TFA for 2 minutes, followed by 100-0% acetonitrile/0.05% TFA and water/0.05% TFA over 3 minutes. The post run time was 2 minutes.

***In vitro* binding assay for bovine/human carbonic anhydrase II:**

General. The fluorescence competition assay developed by Whitesides et al. (J. Am. Chem. Soc. 1994, 116, 5057) and J.C. Kemohan (J. Biol. Chem. 1967, 242, 5813) using DNSA as a reporting ligand that is displaced by the test compound was used for the measurement of binding affinities. The assay is based on the observation that the only fluorescence signal detected at 460 nm upon excitation at 290 nm, an absorption minimum for DNSA, is that of the DNSA•CA complex. With increasing sample concentration the fluorescence intensity due to DNSA•CA decreases as a result of competition with the test compound, allowing the determination of dissociation constants from Scatchard plots. The latter were developed for each test compound using mass balance for calculating the concentrations of CA bound

to DNSA [CA•DNSA], bound to the non-fluorescent sample [CA•Inh] and free CA [CA] in solution based on a dissociation constant Kd for DNSA of 0.39μM (determined by titration experiments) and the total enzyme concentration (0.18 μM). Assay conditions: Increasing amounts of the test compounds (from 10 nM to 10000 nM, stock solutions made in DMSO) were added to a solution of DNSA (20 μM) and bovine or human CA-II (180 nM) in 50mM pH 7.4 potassium phosphate buffer in a 96 well microtiter plate. The solutions were mixed and incubated at room temperature for 1 hr before the changes in fluorescence intensity were determined on a fluorescence plate reader (excitation wavelength at 290 nm and emission wavelength at 460 nm). The Kd values were determined from Scatchard plots using the equation below as described by Whitesides *et.al.*

$$[CA\bullet Inh]/[CA]tot\bullet[Inh] = K\,11inh - K\,11inh\,\{[CA\bullet Inh] + [CA\bullet DNSA]\}/([CA]tot)$$

The terms [CA•DNSA], [CA•Inh], and free CA were calculated using the mass balance based on the known values for the dissociation constant of CA•DNSA and the total concentration of CA in each reaction. The dissociation constants (Kd) of CA•DNSA were determined to be 0.425 μM against human carbonic anhydrase II and 0.393 μM against bovine carbonic anhydrase II, by titrating the respective enzyme (200 nM in pH 7.4 phosphate buffer) with DNSA (from 200 1000 nM, stock solution made in DMSO) and recording the change in fluorescence. These results agree closely with reported values.

Carbonic Anhydrase II

1

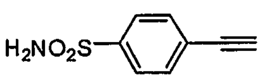

2

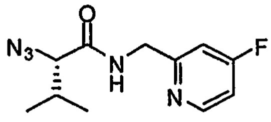

3

Ethoxzolamide (inhibitor)

4

Bovine Serum Albumin

5

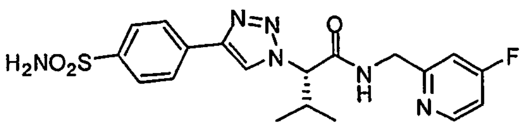

Product

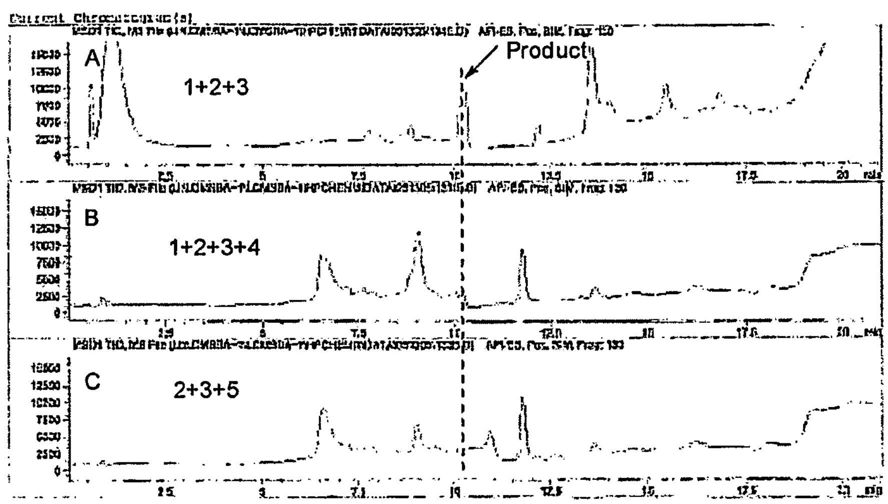


Figures A, B and C are SIM/MS chromatograms of aliquots taken from a typical screen. In this instance, the product was identified as an in situ hit. The binding assay revealed that the compound's Kd was 0.5 nM. Figure A is an aliquot of an incubation mixture of carbonic anhydrase II (1), the anchor molecule (2) and the azide fragment (3). The dotted line represents retention time for the newly formed ligand. Figure B is an aliquot of an incubation mixture of carbonic anhydrase II (1), the anchor molecule (2), the azide fragment (3) and a carbonic anhydrase II inhibitor (4). Figure C is an aliquot of an incubation mixture of bovine serum albumin (5), the anchor molecule (2) and the azide fragment (3). Figure A reveals that the ligand is formed via enzyme templation. Figure B reveals that in the presence of a known inhibitor, the desired ligand is not formed, thus supporting the contention that ligand formation is enzyme templated. And Figure C reveals that in the absence of CA-II, no ligand is formed. This molecular imaging candidate

was choosen for radiolabeling due to the ease of displacement of para-nitro groups by 18F-fluoride in pyridine scaffolds.

General:

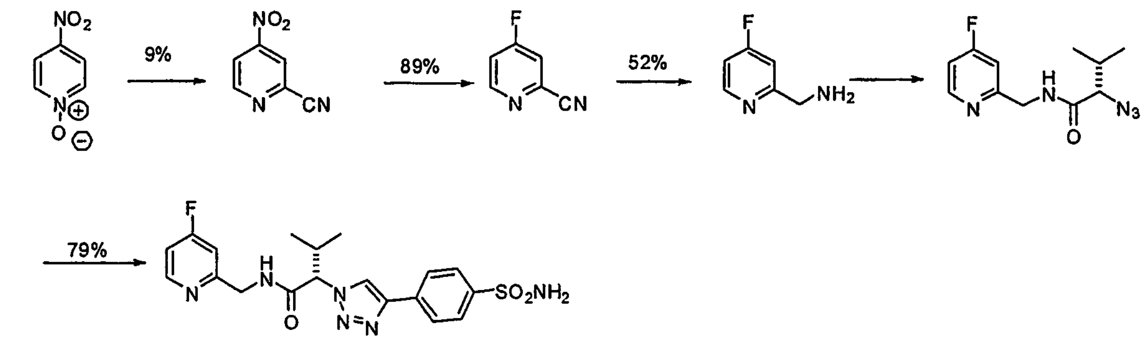


Synthesis of 4-nitro-2-cyano pyridine was carried out according to a known literature procedure (J. Organomet. Chem. 1997, 544, 163-174). Preparation of 4-fluoro-2-cyano pyridine was carried out according to a known literature procedure (Org. Lett. 2005, 7, 577-579).

Synthesis of 4-fluoro-2-aminomethyl pyridine

To a round bottom flask containing 4-fluoro-2-cyano pyridine (278 mg, 2.3 mmol) in THF (10 mL) was added $BH_3$-THF (1M, 6 mL, 6 mmol). The reaction was refluxed for 15 min. The reaction was then cooled to RT and HCl (30 mL) was added with venting. The aqueous layer was washed with $Et_2O$ (3x's). The aqueous layer was made basic with NaOH (15% aq.) and extracted with a minimal volume of $CH_2Cl_2$. The combined organics were washed with brine, dried ($MgSO_4$), filtered and concentrated to dryness in a cold water bath to afford 150 mg of a clear, colorless oil.
$^1$H NMR (300 MHz, $CDCl_3$) δ: 4.0 (2H, s), 6.85-6.95 (1H, m), 7.06 (1H, dd, $J$ = 6.0, 3.0 Hz), 8.46-8.5 (1H, m) $NH_2$ not seen
$^{19}$F NMR (300 MHz, $C_6F_6$) δ: 64.178

Coupling of 4-Fluoro-2-aminomethyl pyridine with L-valineazidoacid

To the L-valineazidoacid (0.038g, 0.268 mM,1eq) in a glass vial dissolved in THF (1mL), stirring at room temperature was added 4-fluoro-2-methylaminopyridine (0.04g, 0.317mM, 1.2 eq), followed by triethylamine (0.243 mL, 1.748 mM, 5 eq), EDC (0.085g, 0.559 mM, 1.6 eq), and HOBt (0.076g, 0.559 mM, 1.6 eq) and left to react for 6 hrs. After the reaction was completed, it was quenched with water and extracted with ethylacetate. Organic layer was washed with water followed by sodium bicarbonate, brine and then dried over $MgSO_4$ After the organic layer was filtered and concentrated to dryness, the reaction mixture was purified by column chromatography using ethylacetate/hexanes as elution solvent (loaded in 80/20 Hex/EtOAc eluted with 100ml, then increased to 50/50 Hex/EtOAc). The product was isolated as a colorless oil (0.021g, 32%).

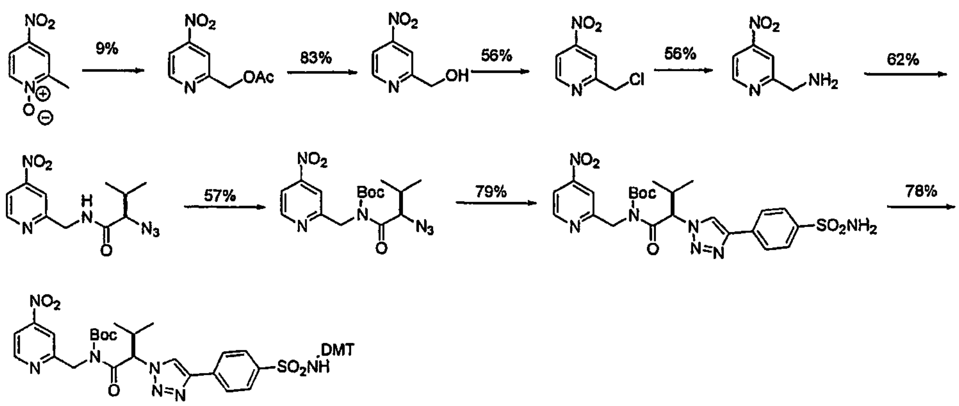

Synthesis of 4-nitro-2-acetoxymethyl pyridine

To a round bottom flask containing acetic anhydride (80 mL) at 90 °C, was added 4-nitro-2-methyl pyridine N-oxide (10 g). The reaction was kept at 120°C overnight. The reaction was then concentrated to dryness. The reaction mixture was purified on silica gel using hexanes (to remove excess acetic anhydride) followed by 30% $Et_2O$:Hex (to remove 4-acetoxy-2-acetoxymethyl pyridine) followed by 50% $Et_2O$:Hex to afford 3.5 g (27.5%) of a yellow solid.
$^1$H NMR (300 MHz, $CDCl_3$) δ: 2.24 (3H, s), 5.37 (2H, s), 7.97-8.00 (1H, m), 8.09 (1H, s), 8.91 (1H, d, *J*= 6.0 Hz)
MS (Electrospray): 197 (M+H), 155 (M-OAc)

Synthesis of 4-nitro-2-hydroxymethyl pyridine

To a round bottom flask containing 4-nitro-2-acetoxtmethyl pyridine (856 mg, 4.4 mmol) was added HCl (1N, 20 mL, 20 mmol). The reaction was heated at 50°C overnight. The reaction then poured onto $Et_2O$. The aqueous layer was washed with $Et_2O$. The aqueous layer was then treated with sat'd $Na_2CO_3$ and extracted with $CH_2Cl_2$ (3x's). The combined organics were dried ($MgSO_4$), filtered and concentrated to dryness to afford 561 mg (83.4%) of a clear, yellow oil.
$^1$H NMR (300 MHz, $CDCl_3$) δ: 2.05-2.55 (1H, br s), 4.95 (2H, s), 7.97 (1H, dd, *J*= 6.0, 3.0 Hz), 8.10 (1H, s), 8.89 (1H, d, *J*= 6.0 Hz)
MS (Electrospray): 155 (M+H)

Synthesis of 4-nitro-2-chloromethyl pyridine

To a round bottom flask containing alcohol (562 mg, 3.65 mmol) and $CH_2Cl_2$ (5 mL) at 0 °C was added $SOCl_2$ (3.65 mL, 7.3 mmol). The reaction was stirred at 0 °C for 2 hrs. The reaction was poured onto sat'd $NaHCO_3$ and extracted into $CH_2Cl_2$. The combined organics were dried ($MgSO_4$), filtered and concentrated to dryness. The reaction mixture was purified on silica gel using 1:1 $Et_2O$:Hex as the eluent to afford 350 mg (55.8%) of a yellow solid.
$^1$H NMR (300 MHz, $CDCl_3$) δ: 4.83 (1H, s), 8.01 (1H, dd, *J*= 6.0, 3.0 Hz), 8.27 (1H, s), 8.90 (1H, d, *J* = 6.0 Hz)

Synthesis of 4-nitro-2-methylamino pyridine

To a round bottom flask containing 4-nitro-2-chloromethyl pyridine (350 mg, 2.03 mmol) was added hexamethylenetetramine (285 mg, 2.03 mmol) and $CHCl_3$ (5 mL). The reaction mixture was heated at reflux overnight. The resultant ppt was filtered off and washed with $Et_2O$ to afford 400 mg of a white solid. The solid was then dissolved in EtOH (7 mL) and treated with HCl (conc, 0.5 mL). The reaction mixture was heated at 90 °C for 2 hrs. The reaction was then concentrated to dryness, poured onto water, washed with $Et_2O$. The aqueous layer was then made basic by adding $Na_2CO_3$. The product was extracted into $CH_2Cl_2$. The combined organics were dried ($MgSO_4$), filtered and concentrated to dryness to afford 175 mg of a clear, colorless oil.
$^1$H NMR (300 MHz, $CDCl_3$) δ: 1.6-1.8 (2H, br s), 4.2 (2H, s), 7.9 (1H, dd, *J*= 6.0, 3.0 Hz), 8.15 (1H, s), 8.83 (1H, d, *J*= 6.0 Hz)
MS (Electrospray): 154 (M+H)

Synthesis of 2-Azido-3-methyl-N-(4-nitro-pyridin-2-ylmethyl)-butyramide

To a round bottom flask containing 4-nitro-2-methylamino pyridine (175 mg, 1.14 mmol) was added THF (5 mL), $Et_3N$ (1.6 mL, 11.4 mmol), 2-Azido-3-methyl-butyryl azide (164 mg, 1.14 mmol), EDC (278 mg, 1.83 mmol) and HOBt (247 mg, 1.83 mmol). The reaction was stirred overnight at RT. The reaction was then poured onto HCl (1N, 20 mL) and extracted into EtOAc. The combined organics were washed with sat'd $NaHCO_3$ dried ($MgSO_4$), filtered and concentrated to dryness to afford 197 mg (61.8%) of a light brown solid.
$^1$H NMR (300 MHz, $CDCl_3$) δ: 0.96 (3H, d, *J*= 6.0 Hz), 1.14 (3H, d, *J*= 6.0 Hz), 2.39-2.50 (1H, m), 3.4-3.7 (1H, br s), 4.76 (2H, d, *J*= 6.0 Hz), 7.97 (1H, dd, *J* = 6.0, 3.0 Hz), 8.02 (1H, s), 8.90 (1H, d, *J*= 6.0 Hz)
MS (Electrospray): 279 (M+H), 301 (M+Na)

Synthesis of (2-Azido-3-methyl-butyryl)-(4-nitro-pyridin-2-ylmethyl)-carbamic acid tert-butyl ester

To a round bottom flask containing the starting azide (400 mg, 1.44 mmol) was added $CH_3CN$ (10 mL), $Boc_2O$ (470 mg, 2.16 mmol) and DMAP (9 mg, 0.07 mmol). The reaction was stirred at RT overnight. The reaction was then poured onto water and extracted into EtOAc. The combined organics were dried ($MgSO_4$), filtered and concentrated to dryness. The residue was purified on silica gel using 2:1 Hex:$Et_2O$ as the eluent to afford 309 mg (57%) of a white solid.
$^1$H NMR (300 MHz, $CDCl_3$) δ: 1.03 (3H, d, *J*= 9 Hz), 1.07 (3H, d, *J*= 6.0 Hz), 1.41 (9H, s), 2.23-2.39 (1H, m), 5.30 (2H, s), 7.89-7.96 (2H, m), 8.81 (1H, d, *J*= 6.0 Hz)

MS (Electrospray): 379 (M+H), 401 (M+Na)

Synthesis of {3-Methyl-2-[4-(4-sulfamoyl-phenyl)-[1,2,3]triazol-1-yl]-butyryl}-(4-nitro-pyridin-2-ylmethyl)-carbamic acid tert-butyl ester

To a round bottom flask containing the azide (300 mg, 0.79 mmol), tBuOH (3 mL), 4-ethyne-benzenesulfonamide (144 mg) was added $CuSO_4$ (0.04 M, 1.5 mL) and sodium ascorbate (0.1 M, 1.2 mL). The reaction was stirred overnight under argon. The reaction was then poured onto water and extracted into EtOAc. The combined organics were washed with 5% $NH_4OH$, dried ($MgSO_4$), filtered and concentrated to dryness. The residue was purified on silica gel first using 30% EtOAc:Hex to elute off the starting reagents followed by 1:1 EtOAc:Hex to afford 350 mg 78.8% of a white solid.
$^1$H NMR (300 MHz, $CDCl_3$) δ: 0.90 (3H, d, *J*= 6.0 Hz), 1.12 (3H, d, *J*= 6.0 Hz), 1.55 (9H, s), 2.59-2.71 (1H, m), 3.40-3.50 (2H, br s), 4.78 (2H, s), 6.87 (1H, d, *J*= 9.0 Hz), 7.88-7.91 (2H, m), 7.98-8.03 (4H, m), 8.28 (1H, s), 8.75 (1H, d, *J*= 6.0 Hz)
MS (Electrospray): 506 (M+H)

Synthesis of {2-[4-(4-{[Bis-(4-methoxy-phenyl)-phenyl-methyl]-sulfamoyl}-phenyl)-[1,2,3]triazol-1-yl]-3-methyl-butyryl}-(4-nitro-pyridin-2-ylmethyl)-carbamic acid tert-butyl ester.

To a round bottom flask containing the triazole (350 mg, 0.63 mmol), CH2Cl2 (5 mL) and TEA (436 uL) was added DMT-Cl (318 mg, 0.94 mmol). The reaction was stirred at RT for 1 hr. TLC (1:1 EtOAc:Hex) indicated complete consumption of starting material. The reaction was then poured onto water and extracted into EtOAc. The combined organics were dried (MgSO4), filtered and concentrated to dryness. The residue was purified on silica gel using 30% EtOAc:Hex to elute off higher running material followed by 40% EtOAc:Hex to afford 417 mg (77%) of a yellow solid.
$^1$H NMR (300 MHz, $CDCl_3$) δ: 0.90 (3H, d, *J*= 6.0 Hz), 1.12 (3H, d, *J*= 6.0 Hz), 1.56 (9H, s), 2.57-2.71 (1H, m), 3.71 (6H, s), 5.75 (2H, s), 6.61-6.71 (2H, m), 6.85 (1H, d, *J*= 9.0 Hz), 7.18-7.26 (5H, m), 7.33-7.37 (1H, m), 7.65 (2H, d, *J*= 9.0 Hz), 7.98-8.03 (2H, m), 8.20 (1H, s), 8.75 (1H, d, *J*= 6.0 Hz)
MS (Electrospray): 862 (M+H)
Radiolabeling of compound

**Carbonic Anhydrase F18 Experimental**

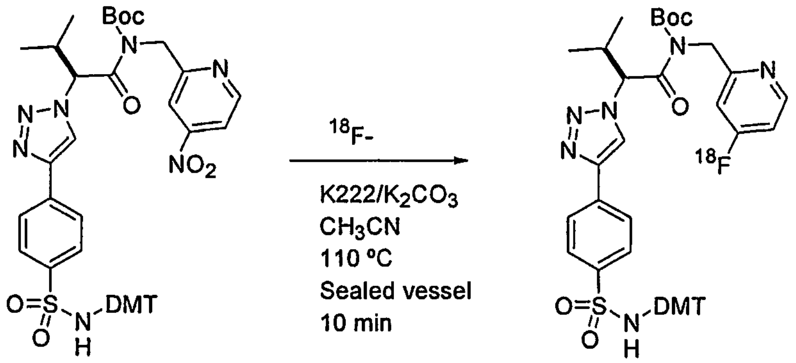


1 2

Oxygen-18 water was irradiated using 11 MeV protons (RDS-111 Eclipse, Siemens Molecular Imaging) to generate [$^{18}$F]fluoride ion in the usual way. At the end of the bombardment, the [$^{18}$O]water containing [$^{18}$F]fluoride ion was transferred from the tantalum target to an automated nucleophilic fluorination module (explora RN, Siemens Biomarker Solutions). Under computer control, the [$^{18}$O]water/[$^{18}$F]fluoride ion solution was transferred to a small anion exchange resin column (Chromafix 45-PS-HCO3, Machery-Nagel) which had previously been rinsed with water (5 mL), aqueous potassium bicarbonate (0.5 *M,* 5 mL), and water (5 mL). The [$^{18}$O]water (1.8 mL) was recovered for subsequent purification and reuse. The trapped [$^{18}$F]fluoride ion was eluted into the reaction vessel with a solution of potassium carbonate (3.0 mg) in water (0.4 mL). A solution of Kryptofix 222 (20 mg) in acetonitrile (1.0 mL) was added, and the mixture was heated (70 to 95°C) under vacuum and a stream of argon to evaporate the acetonitrile and water. After cooling, to the residue of "dry" reactive [$^{18}$F]fluoride ion, K222, and potassium carbonate, was added a solution of 4-nitropyridine-*N*-BOC-valine-*N*-dimethoxytrityl-benzenesulfonamide (**1**, 17 mg, 19.7 μmol) in acetonitrile (1.0 mL). The reaction mixture was heated to 110 °C in a sealed vessel ($P_{max}$ = 2.3 bar) for 10 minutes with stirring (magnetic). The mixture was cooled to 55 °C and most of the acetonitrile was evaporated under vacuum and a stream of argon as before.

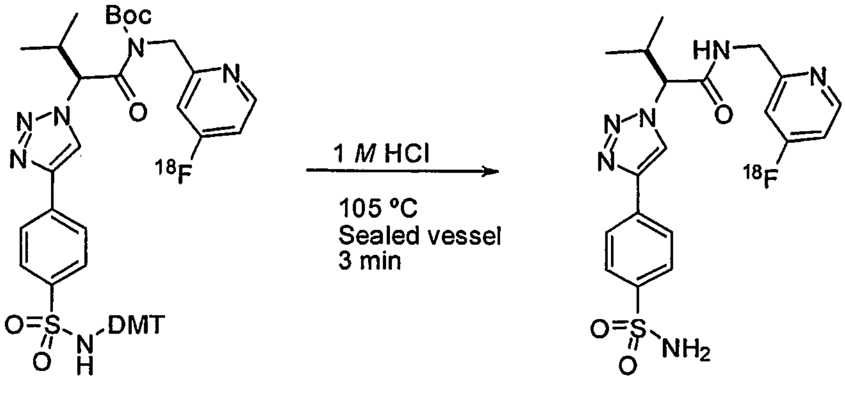


**2** **3**

To the crude protected [$^{18}$F]fluorinated intermediate (**2**) was added aqueous hydrochloric acid (1.0 *M*, 1.0 mL), and the mixture was heated to 105 °C for 3 minutes. After cooling to 35 °C, aqueous sodium acetate (2.0 *M*, 0.5 mL) was added with stirring. The reaction mixture was transferred to a sample loop (1.5 mL), and injected onto a semi-prep HPLC column (Phenomenex Gemini 5μ C18,250 x 10 mm, 25% acetonitrile, 75% water, 0.05% trifluoroacetic acid mobile phase, 5.0 mL/min). The product 4-[$^{18}$F]fluoropyridine-valine-benzenesulfonamide (3, [$^{18}$F]FPVBS) eluted at 15-16 minutes as monitored by flow-through radiation detection and UV (254 nm). The HPLC eluate containing the product (7-8 mL) was collected in a 30 mL vial with a magnetic stir bar, and water (20 mL) was added. The aqueous solution was thoroughly mixed and then passed through a C18 Sep-Pak with the water/mobile phase solution going to a waste bottle. The C18 Sep-Pak was washed with an additional aliquot of water (20 mL). The product was then eluted from the C18 Sep-Pak with ethanol (1.0 mL) which was passed through a 0.22 μm sterile filter into a sterile vial. Water (9.0 mL) was then added to the sterile product vial through the sterile filter to give an ethanol concentration of 10%.

A typical production run starting with about 900 mCi of [$^{18}$F]fluoride ion gave 91 mCi (136 mCi at EOB, 15 % yield) of isolated product after 64 minutes of synthesis, HPLC purification, and C18 solid-phase extraction and reconstitution in 10% ethanol.

The collected product was analyzed by HPLC (Phenomenex Gemini 5μ C18, 150 x 4.6 mm, 25% acetonitrile, 75% water, 0.05% trifluoroacetic acid mobile phase, 1.0 mL/min). As monitored by radioactivity and UV (254 nm) detection, this product had a retention time of 12 minutes and a radiochemical purity of 99.4%.

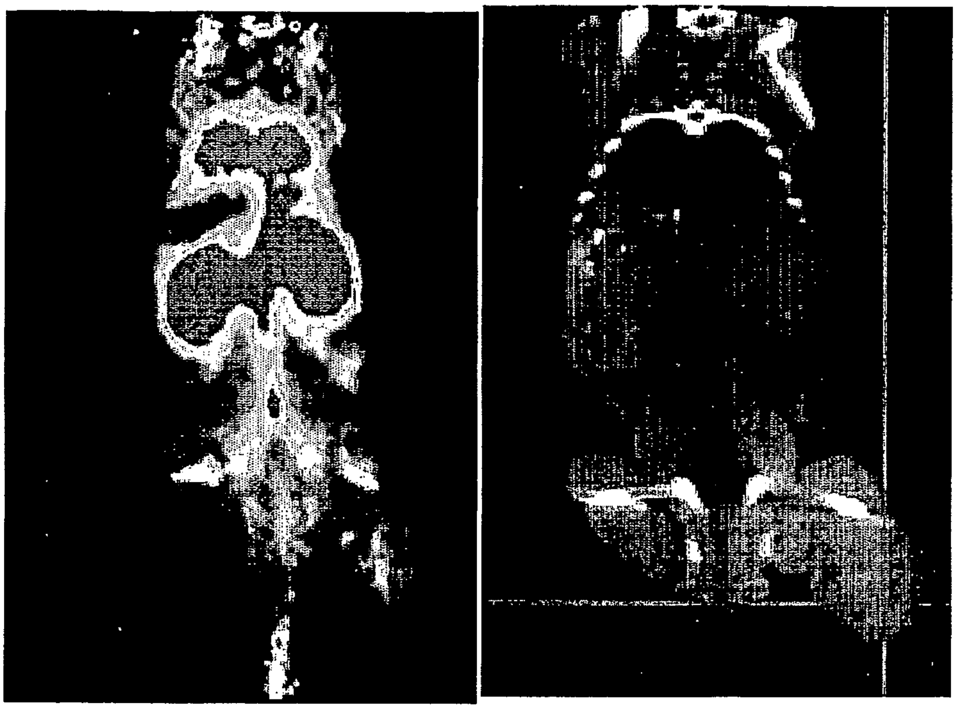

PET IMAGE: 2 hr static PET/CT of a normal mouse.

The 18F-labeled CA-II imaging agent preferentially binds to lungs, kidneys and blood correlating well with their high expression levels of CA-II.

**COX-2 IN SITU SCREENS**

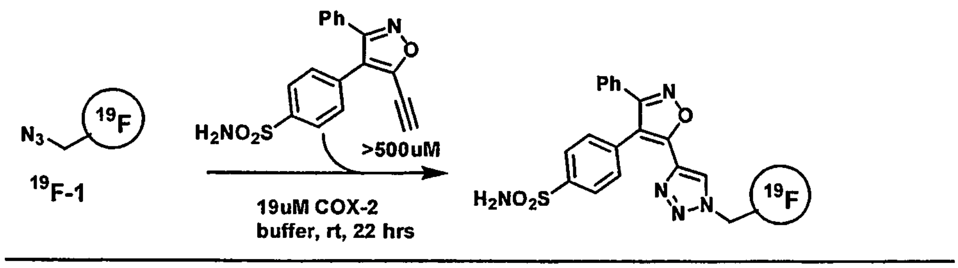


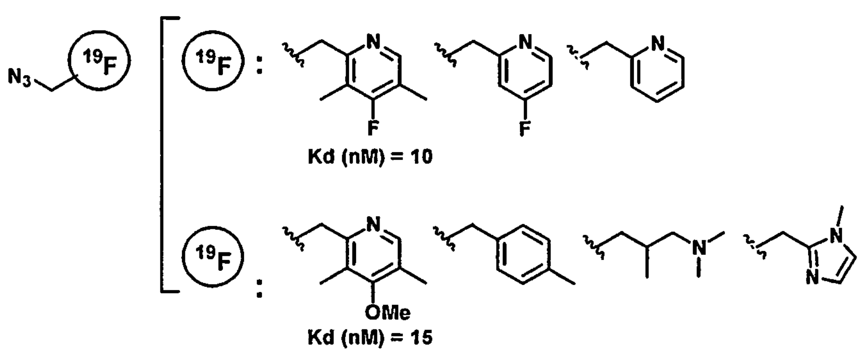


**General**. COX-II human recombinant enzyme isolated from a Baculovirus overexpresssion system in Sf21 cells (Cayman Chemical company, catalog) number 60122, 24,016 Units/mg (diluted to 7μM final concentration in the

reaction) was used for *in situ* click chemistry.

For in vitro activity assay COX-2 colorimetric (Ovine) inhibitor screening assay kit from Cayman Chemical company was used (catalog number 760111). All absorbance measurements were performed on a SPECTRA MAX M2 plate reader at 28°C. The LC/MS analyses were performed on an Agilent 1100 series LC/MSD (SL) using a 30 X 2.1 mm Zorbax C8 column with a Phenomenex C18 pre-column. Compound detection was accomplished by electrospray mass spectroscopy in positive selected ion mode (LC/MS-SIM). The elution solvents, acetonitrile and water, contained 0.05% TFA.

**General procedure for the synthesis of 1,4-disubstituted ("*anti*") triazoles:**

A mixture of alkyne (1eq)) and azide (1eq) in *tert*-butanol (0.400mL) was reacted over night at room temperature in the presence of CuSO4 (0.04M solution in pH 7.4 phosphate buffer, 7.5 mol%), sodium ascorbate (0.1M solution in pH 7.4 phosphate buffer, 15 mol%). The reaction mixture was poured onto water (a few ml) and extracted with ethylacetate, washed with 5% aqueous ammonium hydroxide, dried over $MgSO_4$ and concentrated to yield 98-99% pure triazole as a white solid. The unoptimized yield was 40%. Chromatography was performed on some impure compounds (purity less than 90%) using ethyl acetate:hexane mixture as eluent for purification.

**General procedure for *in situ* click chemistry experiments:**

Stock solutions of azides (20 mM), Valdecoxib (20 mM) and alkyne (3mM) were prepared in DMSO. The alkyne (1 μL) was added to eppendorf tubes (0.5mL volume) containing the enzyme (47.5 μl of commercially available COX-2 human recombinant enzyme from Cayman Chemical company), followed by the azide reagent (1 μL) and DMSO (0.5 μL). The reaction plate was stored at 37 °C for 18-20 hours. The final reagent concentrations were as follows: Enzyme (7 μM), alkyne (60 μM), azide (400 μM) and DMSO (5 vol%).

In parallel, control reactions were set up and subjected to analogous experimental conditions: (1) Competitive inhibition control with valdecoxib (1 μl, 200 μM final concentration); (2) Bovine Serum Albumin control experiments, using 1mg/mL BSA in 100mM TrisHCl (pH 8.0) buffer with 300 μM DDC.

LC/MS-SIM analysis: All samples were analyzed by reverse phase HPLC with electrospray mass spectroscopic detection in the positive selected ion mode. The injection volume was 15 μL at a flow rate of 0.3mL/min. The following elution gradient was employed: 10-100% Acetonitrile/0.05% TFA and water/0.05% TFA over 20 minutes, 100% acetonitrile/0.05%TFA for 2 min followed by 100-10% acetonitrile /0.05% TFA and water/0.05%TFA over 3 mins. The post run time was 3 minutes.

COX-2 Enzyme in 80 mM TrisHCl buffer(pH 8.0) with 0.1% tween and 300μM DDC

1

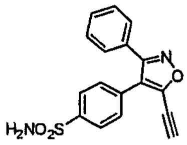

2

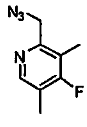

3

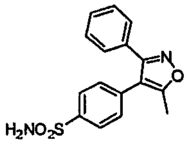

4

Bovine Serum Albumin

5

Cyclo addition product of 2+3 Anti Triazole (Cu reaction)

6

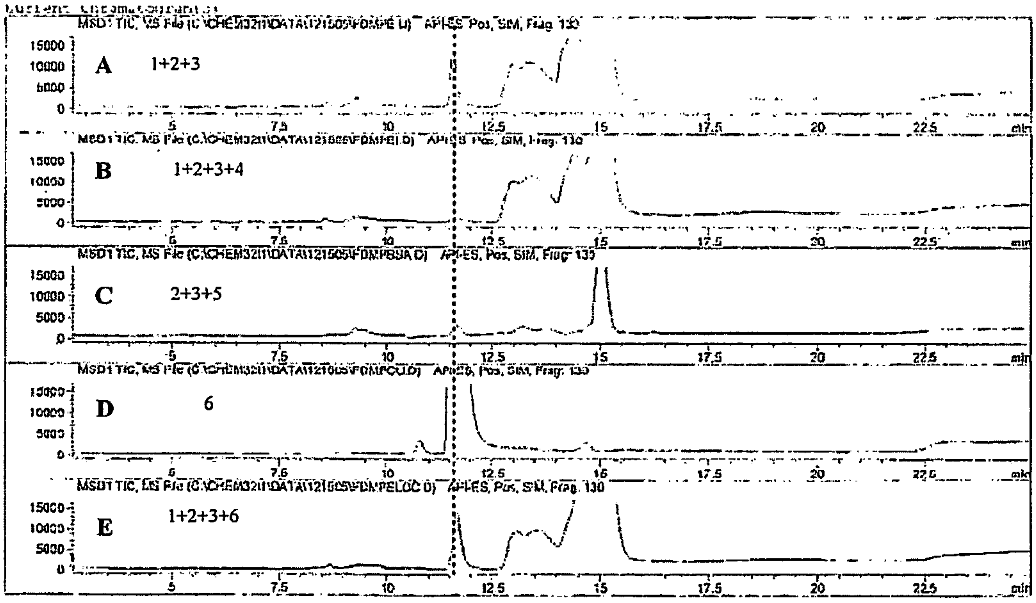


Figures A, B, C, D and E are SIM/MS chromatograms of aliquots taken from a typical screen. In this instance, the product **6** was identified as an in situ hit. The binding assay revealed that the compound's Kd was roughly 10nM. Figure A is an aliquot of an incubation mixture of COX-2 (1), the anchor molecule (2) and the azide fragment (3). The dotted line represents retention time for the newly formed ligand. Figure B is an aliquot of an incubation mixture of COX-2 (1), the anchor molecule (2), the azide fragment (3) and a COX-2 inhibitor, Valdecoxib (4). Figure C is an aliquot of an incubation mixture of bovine serum albumin (5), the anchor molecule (2) and the azide fragment (3). Figure D is the injection of the pure product assembled by coupling (2) and (3) in the presence of Cu(I). Figure E is a co-injection of (6) into the reaction in Figure A to verify the presence of (6). Figure A reveals that the ligand is formed via enzyme templation. Figure B reveals that in the presence of a known inhibitor, the desired ligand is not formed, thus supporting the contention that ligand formation is enzyme templated. And Figure C reveals that in the absence of COX-2, no ligand is formed. This molecular imaging candidate was choosen for radiolabeling due to the ease of displacement of para-nitro groups by 18F-fluoride in pyridine scaffolds.

Synthetic Scheme (continued) for the Precursor:

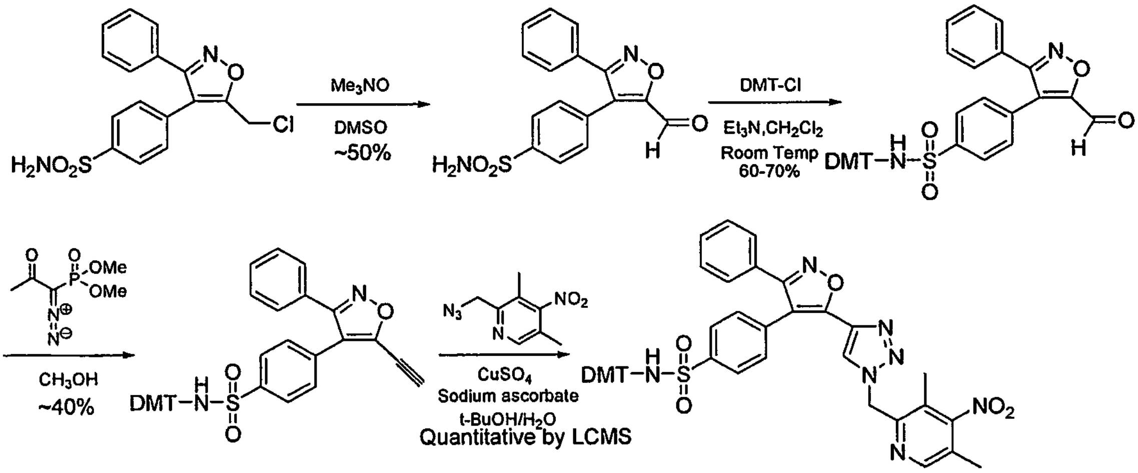


Experimental Procedures:

The chloromethyl sulfonamide was prepared according to literature procedures (see: Talley, J. J. et al. J. Med. Chem. 2000, 43, 775-777).

Sulfonamide Aldehyde: Trimethyl-N-oxide (1.72g, 22.98 mM, 4eq) was added to the chlorosulfonamide (2.00g, 5.74 mM) in DMSO (10mL) stirring under argon in a round bottom flask. The reaction was quenched with water after 1hr when no starting material was seen on TLC. The reaction mixture was then extracted with ethyl acetate and the organic layer was washed 2 times with water, dried over $MgSO_4$, filtered and concentrated to dryness. The crude isolated product was off-white powder (~ 1.00g, 50% yield) and was used as it is in the next step.

DMT protected aldehyde: Triethyl amine (0.56 mL, 3.99 mM, 4 eq) was added to the sulfonamide aldehyde (0.328 g, 0.998 mM) in methylene chloride (15mL) stirring under argon followed by DMT-chloride (0.44 g, 1.29 mM, 1.3 eq) and was left to react for 18 hrs. The reaction mixture was then quenched with water and extracted with methylene chloride followed by washing the organic layer with brine and dried over $MgSO_4$. After the organic layer was filtered and concentrated to dryness, the product was purified by chromatography over silica gel with ethylacetate/hexane mixture as a eluent. The isolated product was a light yellow colored powder (0.44 g, 70%).

DMT protected alkyne: To a mixture of tosyl azide (0.304 g, 1.54 mM) and potassium carbonate (0.32 g, 2.31 mM) stirring in acetonitrile (20 mL) under argon, was added phosphonate reagent (0.128 g, 0.77 mM). The reaction turned cloudy gradually. After 2 hrs, the aldehyde (0.407 g, 0.65 mM), dissolved in methanol:acetonitrile (20mL:20mL), was added slowly and the heterogeneous reaction mixture became yellow homogeneous solution over time. After 6 hrs the reaction mixture was quenched with water and extracted with ethylacetate, washed with water and dried over $MgSO_4$. After filtration and concentration to dryness, the product was purified by column chromatography and obtained the alkyne as a white foamy compound (0.2g, 50%).

The triazole was synthesized using the general procedure for the 1,4-disubstituted triazoles as described in general procedures for making the anti triazoles.

Preparation of the 18F-labeled COX-2 imaging agent

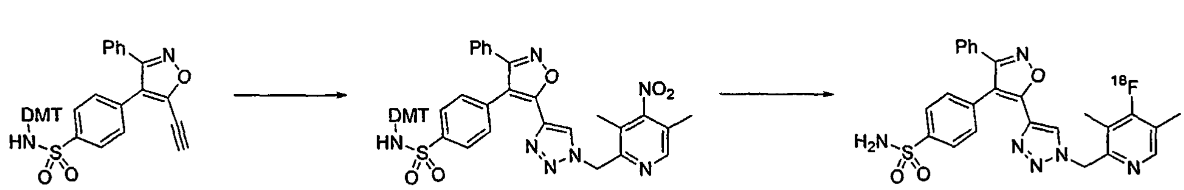

Synthesis of N-[Bis-(4-methoxy-phenyl)-phenyl-methyl]-4-{5-[1-(3,5-dimethyl-4-nitro-pyridin-2-ylmethyl)-1H-[1,2,3]triazol-4-yl]-3-phenyl-isoxazol-4-yl}-benzenesulfonamide

To a round bottom flask containing the alkyne (157 mg, 0.25 mmol) was added tBuOH (10 mL), $CuSO_4$ (0.04M, 624uL), sodium ascorbate (0.1M, 500 uL) and the azide (52 mg, 0.25 mmol). The heterogeneous solution was stirred overnight at RT. The reaction was poured onto EtOAc and washed with dilute $NH_4OH$ and water. The organics were dried ($MgSO_4$), filtered and concentrated to dryness. The residue was purified on silica gel (packed using 1:1 EtOAc: Hex) first by loading with $CH_2Cl_2$ and then eluting with 1:1 EtOAc:Hex to afford 115 mg (55%) of a white solid.
$^1$H NMR (300 MHz, DMSO-$d_6$) δ: 2.21 (3H, s), 2.27 (3H, s), 3.64 (6H, s), 5.95 (2H, s), 6.67-6.70 (4H, m), 7.09-7.52 (18H, m), 8.42 (1H, s), 8.58 (1H, s), 8.65 (1H, s).
LC/MS: Calc'd for $C_{46}H_{39}N_7O_7S$: 833.26; found: 834.2 (M+H)

2-Azidomethyl-3,5-dimethyl-4-nitro-pyridine

To a round bottom flask containing 4-nitro-1-hydroxymethyl-3,5-dimethylpyridine (0.91 g, 5 mmol) in $CH_2Cl_2$ (10mL) was added triethylamine (558 uL, 8 mmol) and p-toluenesulfonic anhydride (1.96 g, 6 mmol). The reaction was stirred at RT for 2 hrs. The reaction was poured onto water and extracted into $CH_2Cl_2$. The organics were dried ($MgSO_4$), filtered and concentrated to dryness. The solid was redissolved in MeOH (25 mL) and treated with $NaN_3$ (390 mg, 6 mmol) predissolved in water (5mL). The reaction was stirred at RT for 4 hrs. The reaction was then poured onto water and extracted into $CH_2Cl_2$. The organics were dried ($MgSO_4$), filtered and concentrated to dryness. The solid was purified on silica gel using 40% $Et_2O$:Hex as the eluent to afford 600 mg (58%) of a clear, colorless oil.
$^1$H NMR (300 MHz, $CDCl_3$) δ: 2.31 (3H, s), 2.33 (3H, s), 4.51 (2H, s), 8.53 (1H, s)
LC/MS: Calc'd for $C_8H_9N_5O_2$: 207.08; found: 180.2 (M+H-$N_2$), 208.2 (M+H)

COX-2 Click F-18 Experimental

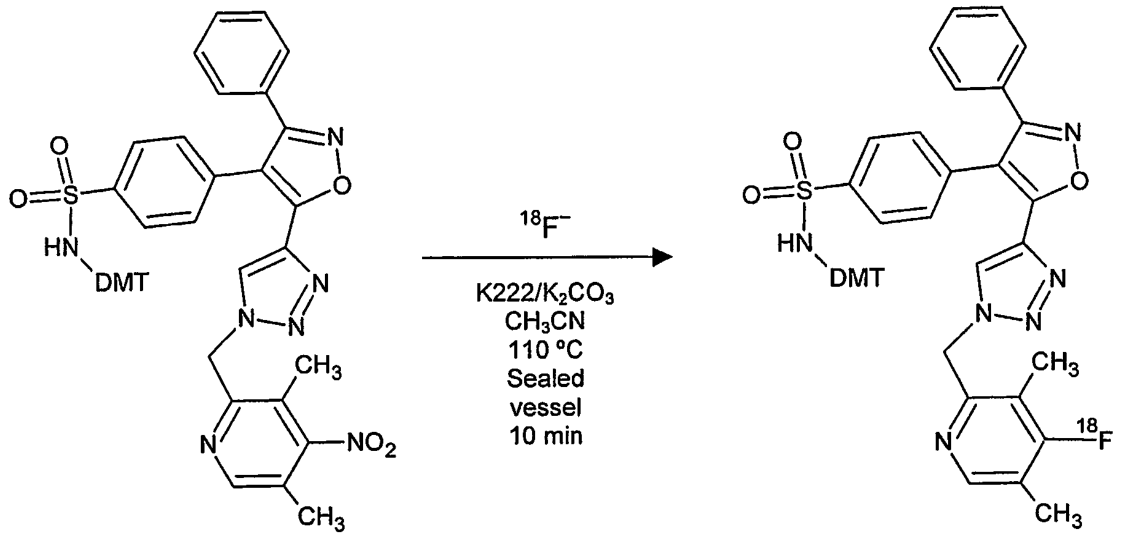


1 2

Oxygen-18 water was irradiated using 11 MeV protons (RDS-111 Eclipse, Siemens Molecular Imaging) to generate [$^{18}$F]fluoride ion in the usual way. At the end of the bombardment, the [$^{18}$O]water containing [$^{18}$F]fluoride ion was transferred from the tantalum target to an automated nucleophilic fluorination module (*explora RN,* Siemens Biomarker Solutions). Under computer control, the [$^{18}$O]water/[$^{18}$F]fluoride ion solution was transferred to a small anion exchange resin column (Chromafix 45-PS-HCO3, Machery-Nagel) which had previously been rinsed with water (5 mL), aqueous potassium bicarbonate (0.5 *M,* 5 mL), and water (5 mL). The [$^{18}$O]water (1.8 mL) was recovered for subsequent purification and reuse. The trapped [$^{18}$F]fluoride ion was eluted into the reaction vessel with a solution of potassium carbonate (3.0 mg) in water (0.4 mL). A solution of Kryptofix 222 (20 mg) in acetonitrile (1.0 mL) was added, and the

mixture was heated (70 to 95 °C) under vacuum and a stream of argon to evaporate the acetonitrile and water. After cooling, to the residue of "dry" reactive [$^{18}$F]fluoride ion, K222, and potassium carbonate, was added a solution of 4-{5-[1-(4-nitro-3,5-dimethyl-pyridin-2-ylmethyl)-1*H*-[1,2,3]triazol-4-yl]-3-phenyl-isoxazol-4-yl}-*N*-dimethoxytrityl-benzenesulfonamide (1, 8.0 mg, 9.6 μmol) in acetonitrile (800 μL). The reaction mixture was heated to 110 °C in a sealed vessel ($P_{max}$ = 2.3 bar) for 10 minutes with stirring (magnetic). The mixture was cooled to 55°C and most of the acetonitrile was evaporated under vacuum and a stream of argon as before.

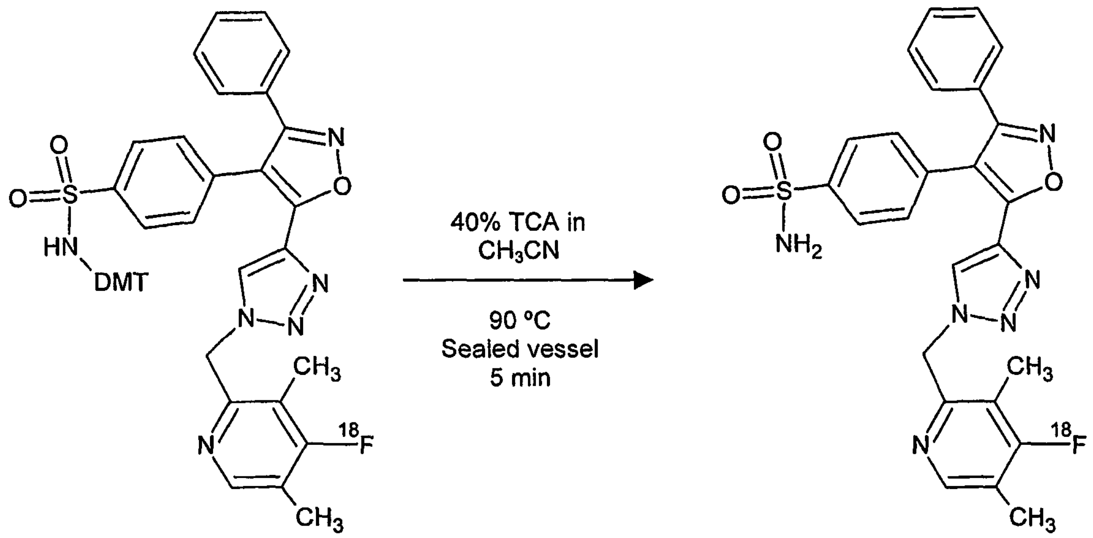


2 3

To the crude protected [$^{18}$F]fluorinated intermediate (**2**) was added a 40% solution of trichloroacetic acid in acetonitrile (600 μL), and the mixture was heated to 90°C for 5 minutes. After cooling to 35 °C, aqueous sodium acetate (2.0 *M*, 550 μL) was added with stirring. The reaction mixture was transferred to a sample loop (1.5 mL), and injected onto a semi-prep HPLC column (Phenomenex Gemini 5μ C18,250 x 10 mm, 55% acetonitrile, 45% water, 0.01% trifluoroacetic acid mobile phase, 5.0 mL/min). The product 4-{5-[1-(4-[$^{18}$F]fluoro-3,5-dimethylpyridin-2-ylmethyl)-1*H*-[1,2,3]triazol-4-yl]-3-phenyl-isoxazol-4-yl}-benzenesulfonamide (3, 5-[1-(4-[$^{18}$F]fluoro-3,5-dimethyl-pyridin-2-ylmethyl) 1*H*-[1,2,3]triazol-4-yl]-valdecoxib, [$^{18}$F]FPVC) eluted at 8.5-9.5 minutes as monitored by flow-through radiation detection and UV (254 nm). The HPLC eluate containing the product (7-8 mL) was collected in a 30 mL vial with a magnetic stir bar, and water (20 mL) was added. The aqueous solution was thoroughly mixed and then passed through a C18 Sep-Pak with the water/mobile phase solution going to a waste bottle. The C18 Sep-Pak was washed with an additional aliquot of water (20 mL). The product was then eluted from the C18 Sep-Pak with ethanol (1.0 mL) which was passed through a 0.22 μm sterile filter into a sterile vial. Water (9.0 mL) was then added to the sterile product vial through the sterile filter to give an ethanol concentration of 10%.

A typical production run starting with about 660 mCi of [$^{18}$F]fluoride ion gave 69.3 mCi (96.2 mCi at EOB, 14.6 % yield) of isolated product after 52 minutes of synthesis, HPLC purification, and C18 solid-phase extraction and reconstitution in 10% ethanol.

The collected product was analyzed by HPLC (Phenomenex Gemini 5μ C18, 150 x 4.6 mm, 55% acetonitrile, 475% water, 0.01% trifluoroacetic acid mobile phase, 1.0 mL/min). As monitored by radioactivity and UV (254 nm) detection, this product had a retention time of 6.1 minutes and a radiochemical purity of 99.9%.

## Claims

1. A method for identifying a candidate imaging probe, the method comprising:

   a) contacting a first library of candidate compounds with a target biomacromolecule, each compound of the first library of compounds comprises a first functional group capable of participating in a click chemistry reaction, and each compound optionally exhibiting affinity for a first binding site of the target biomacromolecule that comprises the first binding site and a second binding site;

b) identifying a first member from the first library of candidate compounds exhibiting affinity for the first binding site;
c) contacting the first member identified from the first library of candidate compounds exhibiting affinity for the first binding site with the target biomacromolecule;
d) contacting a second library of candidate compounds with the first member and the target biomacromolecule, the second library of candidate compounds optionally exhibiting affinity for the second binding site, wherein each compound of the second library of candidate compounds comprises a complementary second functional group capable of participating in a click chemistry reaction with the first functional group,
wherein either each compound of the first library or each compound of the second library, or each compound of both the first and the second library of compounds, independently comprises a non-radioactive isotope of a chemical element and wherein the chemical element comprises at least one radioisotope, optionally attached via a linker, wherein the non-radioactive isotope is F-19, and wherein the radioactive isotope is F-18;
e) reacting the complementary second functional group with the first functional group via a biomacromolecule induced click chemistry reaction to form the candidate imaging probe;
f) isolating and identifying the candidate imaging probe;
g) preparing the candidate imaging probe by chemical synthesis; and
h) converting the candidate imaging probe into an imaging probe by converting the non-radioactive isotope of the element into the radioactive isotope F-18.

**2.** The method of Claim 1, wherein the biomacromolecule is selected from the group consisting of enzymes, receptors, DNA, RNA, ion channels and antibodies.

**3.** The method of Claim 1, wherein the target biomacromolecule is a protein that is overexpressed in disease states, including beta-amyloid in brain tissue of Alzheimer's Disease patients.

**4.** The method of Claim 1, wherein the second binding site constitutes a portion of the first binding site.

**5.** The method of Claim 1, wherein each compound of the first library or each compound of the second library, or each compound of both the first and the second library of compounds comprises a fluorophore.

**6.** The method of Claim 1, wherein the click chemistry reaction is a cycloaddition reaction.

**7.** The method of Claim 6, wherein the cycloaddition reaction is selected from the group consisting of a Diels-Alder reaction or a 1,3-dipolar cycloaddition reaction.

**8.** The method of Claim 7, wherein the cycloaddition reaction is a 1,3-dipolar cycloaddition reaction.

**9.** The method of Claim 1, wherein the complementary click functional groups comprise an azide and an alkyne and wherein the click reaction forms a product comprising 1,2,3 triazole.

**10.** The method of Claim 1, wherein the first functional group is an azide and the second functional group is an alkyne, or wherein the first functional group is an alkyne and the second functional group is an azide.

**11.** The method of Claim 10, wherein the alkyne is a terminal alkyne.

**12.** The method of Claim 1, wherein the steps of a) to f) are performed in an iterative procedure of preparing a new first library of compounds and/or second library of compounds and re-screening until a candidate imaging probe with optimized binding, biodistribution, metabolism and pharmacokinetic properties is identified.

**13.** The method of Claim 12, wherein the identified candidate imaging probe is labeled with the radioactive isotope, and the resulting radioactive imaging probe is used for an imaging method selected from the group consisting of PET, SPECT and optical imaging.

**14.** The method of Claim 1, wherein the target biomacromolecule is an enzyme.

**15.** The method of Claim 14, wherein the target enzyme is selected from the group consisting of overexpressed or overactivated in disease states such as COX-2, AKT, P13K, or CA-9/CA-12.

**16.** The method of Claim 14, wherein the first functional group is an azide and the second functional group is a terminal

alkyne, or wherein the first functional group is a terminal alkyne and the second functional group is an azide.

**17.** The method of Claim 14, wherein the complementary click functional groups comprises an azide and an alkyne and the click reaction forms a 1,2,3 triazole comprising product.

**18.** The method of Claim 14, wherein the first functional group is an azide and the second functional group is an alkyne, or wherein the first functional group is an alkyne and the second functional group is an azide.

**19.** The method of Claim 14, wherein the steps of a) to f) are performed in an iterative procedure of preparing a new first library of compounds and/or second library of compounds and re-screening until a candidate imaging probe with optimized binding, biodistribution, metabolism and pharmacokinetic properties is identified.

**20.** The method of Claim 14, wherein the identified ligand compound is labeled with the radioactive isotope, and the resulting ligand compound is used for an imaging methods selected from the group consisting of PET, SPECT and optical imaging.

**21.** The method of Claim 14, wherein the binding of the candidate compounds within the enzyme binding sites facilitates the click chemistry reaction in the absence of any externally added catalyst.

**22.** The method of Claim 14, wherein the first library of candidate compounds and/or the second library of candidate compounds further comprises a linker between the compound and the first functional group and/or a linker between the compound and the second functional group.

**23.** The method of Claim 22, wherein the linker comprises between 1 to 10 atoms in the linker chain between the compound and the functional group.

**Patentansprüche**

**1.** Verfahren zum Identifizieren einer Kandidat-Abbildungssonde, wobei das Verfahren umfasst:

a) Kontaktieren einer ersten Bibliothek von Kandidatverbindungen mit einem Ziel-Biomakromolekül, wobei jede Verbindung der ersten Bibliothek von Verbindungen eine erste funktionelle Gruppe umfasst, die in der Lage ist, an einer Click-Chemie-Reaktion teilzunehmen, und jede Verbindung optional eine Affinität zu einer ersten Bindungsstelle des Ziel-Biomakromoleküls aufweist, welches die erste Bindungsstelle und eine zweite Bindungsstelle umfasst;
b) Identifizieren eines ersten Mitglieds aus der ersten Bibliothek von Kandidatverbindungen, welches eine Affinität zu der ersten Bindungsstelle aufweist;
c) Kontaktieren des ersten Mitglieds, das aus der ersten Bibliothek von Kandidatverbindungen identifiziert wurde und eine Affinität zu der ersten Bindungsstelle aufweist, mit dem Ziel-Biomakromolekül;
d) Kontaktieren einer zweiten Bibliothek von Kandidatverbindungen mit dem ersten Mitglied und dem Ziel-Biomakromolekül, wobei die zweite Bibliothek von Kandidatverbindungen optional eine Affinität zu der zweiten Bindungsstelle aufweist, wobei jede Verbindung der zweiten Bibliothek von Kandidatverbindungen eine komplementäre zweite funktionelle Gruppe umfasst, die in der Lage ist, an einer Click-Chemie-Reaktion mit der ersten funktionellen Gruppe teilzunehmen,
wobei entweder jede Verbindung der ersten Bibliothek oder jede Verbindung der zweiten Bibliothek oder jede Verbindung sowohl der ersten als auch der zweiten Bibliothek von Verbindungen unabhängig ein nicht radioaktives Isotop eines chemischen Elements umfasst und wobei das chemische Element mindestens ein Radioisotop umfasst, das optional über einen Linker gebunden ist, wobei das nicht radioaktive Isotop F-19 ist und wobei das radioaktive Isotop F-18 ist;
e) Umsetzen der komplementären zweiten funktionellen Gruppe mit der ersten funktionellen Gruppe über eine durch ein Biomakromolekül induzierte Click-Chemie-Reaktion, um die Kandidat-Abbildungssonde zu bilden;
f) Isolieren und Identifizieren der Kandidat-Abbildungssonde;
g) Herstellen der Kandidat-Abbildungssonde durch chemische Synthese; und
h) Umwandeln der Kandidat-Abbildungssonde in eine Abbildungssonde durch Umwandeln des nicht radioaktiven Isotops des Elements in das radioaktive Isotop F-18.

**2.** Verfahren nach Anspruch 1, wobei das Biomakromolekül aus der Gruppe ausgewählt ist, die aus Enzymen, Rezeptoren, DNA, RNA, Ionenkanälen und Antikörpern besteht.

**3.** Verfahren nach Anspruch 1, wobei das Ziel-Biomakromolekül ein Protein ist, welches in Krankheitszuständen überexprimiert ist, darunter Beta-Amyloid im Hirngewebe von Patienten, die an der Alzheimer-Krankheit leiden.

**4.** Verfahren nach Anspruch 1, wobei die zweite Bindungsstelle einen Abschnitt der ersten Bindungsstelle darstellt.

**5.** Verfahren nach Anspruch 1, wobei jede Verbindung der ersten Bibliothek oder jede Verbindung der zweiten Bibliothek oder jede Verbindung sowohl der ersten als auch der zweiten Bibliothek von Verbindungen ein Fluorophor umfasst.

**6.** Verfahren nach Anspruch 1, wobei die Click-Chemie-Reaktion eine Cycloadditionsreaktion ist.

**7.** Verfahren nach Anspruch 6, wobei die Cycloadditionsreaktion aus der Gruppe ausgewählt ist, die aus einer Diels-Alder-Reaktion und einer 1,3-dipolaren Cycloadditionsreaktion besteht.

**8.** Verfahren nach Anspruch 7, wobei die Cycloadditionsreaktion eine 1,3-dipolare Cycloadditionsreaktion ist.

**9.** Verfahren nach Anspruch 1, wobei die komplementären funktionellen Gruppen der Click-Reaktion ein Azid und ein Alkin umfassen, und wobei die Click-Reaktion ein Produkt bildet, das 1,2,3-Triazol umfasst.

**10.** Verfahren nach Anspruch 1, wobei die erste funktionelle Gruppe ein Azid ist und die zweite funktionelle Gruppe ein Alkin ist oder wobei die erste funktionelle Gruppe ein Alkin ist und die zweite funktionelle Gruppe ein Azid ist.

**11.** Verfahren nach Anspruch 10, wobei das Alkin ein terminales Alkin ist.

**12.** Verfahren nach Anspruch 1, wobei die Schritte a) bis f) in einer iterativen Prozedur des Herstellens einer neuen ersten Bibliothek von Verbindungen und/oder zweiten Bibliothek von Verbindungen und des erneuten Screenings durchgeführt werden, bis eine Kandidat-Abbildungssonde mit optimierter Bindung und Bioverteilung, optimiertem Metabolismus und optimierten pharmakokinetischen Eigenschaften identifiziert ist.

**13.** Verfahren nach Anspruch 12, wobei die identifizierte Kandidat-Abbildungssonde mit dem radioaktiven Isotop markiert wird und die resultierende radioaktive Abbildungssonde für ein Abbildungsverfahren verwendet wird, das aus der Gruppe ausgewählt ist, die aus PET, SPECT und optischer Bildgebung besteht.

**14.** Verfahren nach Anspruch 1, wobei das Ziel-Biomakromolekül ein Enzym ist.

**15.** Verfahren nach Anspruch 14, wobei das Ziel-Enzym aus der Gruppe ausgewählt ist, die aus in Krankheitszuständen überexprimierten oder überaktivierten Enzymen wie COX-2, AKT, P13K oder CA-9/CA-12 besteht.

**16.** Verfahren nach Anspruch 14, wobei die erste funktionelle Gruppe ein Azid und die zweite funktionelle Gruppe ein terminales Alkin ist oder wobei die erste funktionelle Gruppe ein terminales Alkin und die zweite funktionelle Gruppe ein Azid ist.

**17.** Verfahren nach Anspruch 14, wobei die komplementären funktionellen Gruppen der Click-Reaktion ein Azid und ein Alkin umfassen und wobei die Click-Reaktion ein Produkt bildet, das 1,2,3-Triazol umfasst.

**18.** Verfahren nach Anspruch 14, wobei die erste funktionelle Gruppe ein Azid und die zweite funktionelle Gruppe ein Alkin ist oder wobei die erste funktionelle Gruppe ein Alkin und die zweite funktionelle Gruppe ein Azid ist.

**19.** Verfahren nach Anspruch 14, wobei die Schritte a) bis f) in einer iterativen Prozedur des Herstellens einer neuen ersten Bibliothek von Verbindungen und/oder zweiten Bibliothek von Verbindungen und des erneuten Screenings durchgeführt werden, bis eine Kandidat-Abbildungssonde mit optimierter Bindung und Bioverteilung, optimiertem Metabolismus und optimierten pharmakokinetischen Eigenschaften identifiziert ist.

**20.** Verfahren nach Anspruch 14, wobei die identifizierte Ligandenverbindung mit dem radioaktiven Isotop markiert wird und die resultierende Ligandenverbindung für ein Abbildungsverfahren verwendet wird, das aus der Gruppe ausgewählt ist, die aus PET, SPECT und optischer Bildgebung besteht.

**21.** Verfahren nach Anspruch 14, wobei die Bindung der Kandidatverbindungen innerhalb der Enzymbindungsstellen die Click-Chemie-Reaktion bei Nichtvorhandensein eines extern zugesetzten Katalysators erleichtert.

**22.** Verfahren nach Anspruch 14, wobei die erste Bibliothek von Kandidatverbindungen und/oder die zweite Bibliothek von Kandidatverbindungen ferner einen Linker zwischen der Verbindung und der ersten funktionellen Gruppe und/ oder einen Linker zwischen der Verbindung und der zweiten funktionellen Gruppe umfasst.

**23.** Verfahren nach Anspruch 22, wobei der Linker 1 bis 10 Atome in der Linkerkette zwischen der Verbindung und der funktionellen Gruppe umfasst.

## Revendications

**1.** Procédé pour identifier un candidat-sonde d'imagerie, lequel procédé comprend :

a) la mise en contact d'une première bibliothèque de composés candidats avec une biomacromolécule cible, chaque composé de la première bibliothèque de composés comprenant un premier groupement fonctionnel capable de participer à une réaction de chimie "clic" et chaque composé présentant facultativement une affinité avec un premier site de liaison de la biomacromolécule cible comprenant le premier site de liaison et un deuxième site de liaison ;
b) identification d'un premier membre de la première bibliothèque de composés candidats présentant une affinité avec le premier site de liaison ;
c) mise en contact du premier membre identifié dans la première bibliothèque de composés candidats présentant une affinité avec le premier site de liaison avec la biomacromolécule cible ;
d) mise en contact d'une deuxième bibliothèque de composés candidats avec le premier membre et la biomacromolécule cible, la deuxième bibliothèque de composés candidats présentant facultativement une affinité avec le deuxième site de liaison, chaque composé de la deuxième bibliothèque de composés candidats comprenant un deuxième groupement fonctionnel complémentaire capable de participer à une réaction de chimie clic avec le premier groupement fonctionnel,
dans lequel chaque composé de la première bibliothèque ou chaque composé de la deuxième bibliothèque, ou chaque composé de la première bibliothèque de composés et de la deuxième comprend indépendamment un isotope non radioactif d'un élément chimique et dans lequel l'élément chimique comprend au moins un isotope radioactif, relié facultativement par une liaison, l'isotope non radioactif étant $F^{19}$ et l'isotope radioactif étant $F^{18}$ ;
e) mise en réaction du deuxième groupement fonctionnel complémentaire avec le premier groupement fonctionnel à l'aide d'une réaction de chimie clic induite par la biomacromolécule pour former le candidat-sonde d'imagerie ;
f) isolement et identification du candidat-sonde d'imagerie ;
g) préparation du candidat-sonde d'imagerie par synthèse chimique ; et
h) conversion du candidat-sonde d'imagerie en une sonde d'imagerie par la conversion de l'isotope non radioactif de l'élément en l'isotope radioactif $F^{18}$.

**2.** Procédé selon la revendication 1, dans lequel la biomacromolécule est sélectionnée dans le groupe comprenant des enzymes, des récepteurs, l'ADN, l'ARN, des canaux ioniques et des anticorps.

**3.** Procédé selon la revendication 1, dans lequel la biomacromolécule cible est une protéine qui est surexprimée dans les états pathologiques, notamment la bêta-amyloïde dans les tissus cérébraux des patients atteints de la maladie d'Alzheimer.

**4.** Procédé selon la revendication 1, dans lequel le deuxième site de liaison constitue une partie du premier site de liaison.

**5.** Procédé selon la revendication 1, dans lequel chaque composé de la première bibliothèque ou chaque composé de la deuxième bibliothèque ou chaque composé de la première bibliothèque de composés et de la deuxième comprend un fluorophore.

**6.** Procédé selon la revendication 1, dans lequel la réaction de chimie clic est une réaction de cycloaddition.

7. Procédé selon la revendication 6, dans lequel la réaction de cycloaddition est sélectionnée dans le groupe comprenant la réaction de Diels-Alder et une réaction de cycloaddition 1,3-dipolaire.

8. Procédé selon la revendication 7, dans lequel la réaction de cycloaddition est une réaction de cycloaddition 1,3-dipolaire.

9. Procédé selon la revendication 1 dans lequel les groupements fonctionnels complémentaires en clic comprennent un azoture et un alkyne et dans lequel la réaction clic forme un produit contenant du 1,2,3-triazole.

10. Procédé selon la revendication 1, dans lequel le premier groupement fonctionnel est un azoture et le deuxième groupement fonctionnel est un alkyne ou dans lequel le premier groupement fonctionnel est un alkyne et le deuxième groupement fonctionnel est un azoture.

11. Procédé selon la revendication 10, dans lequel l'alkyne est un alkyne terminal.

12. Procédé selon la revendication 1, dans lequel les étapes de a) à f) sont réalisées selon une procédure itérative de préparation d'une nouvelle première bibliothèque de composés et/ou d'une nouvelle deuxième bibliothèque de composés et de nouveau tri jusqu'à ce qu'un candidat-sonde d'imagerie ayant des propriétés optimisées de liaison, de biodistribution, métaboliques et pharmacocinétiques soit identifié.

13. Procédé selon la revendication 12, dans lequel le candidat-sonde d'imagerie identifié est marqué avec l'isotope radioactif et la sonde d'imagerie radioactive en résultant est utilisée pour une méthode d'imagerie choisie parmi le groupe comprenant les méthodes d'imagerie PET, SPECT et optique.

14. Procédé selon la revendication 1, dans lequel la biomacromolécule cible est un enzyme.

15. Procédé selon la revendication 14, dans lequel l'enzyme cible est choisi parmi le groupe comprenant des enzymes surexprimés ou suractivés dans les états pathologiques, tels que COX-2, AKT, P13K ou CA-9/CA-12.

16. Procédé selon la revendication 14, dans lequel le premier groupement fonctionnel est un azoture et le deuxième groupement fonctionnel est un alkyne terminal, ou dans lequel le premier groupement fonctionnel est un alkyne terminal et le deuxième groupement fonctionnel est un azoture.

17. Procédé selon la revendication 14, dans lequel les groupements fonctionnels complémentaires en clic comprennent un azoture et un alkyne et la réaction clic donne un produit contenant du 1,2,3-triazole.

18. Procédé selon la revendication 14, dans lequel le premier groupement fonctionnel est un azoture et le deuxième groupement fonctionnel est un alkyne, ou dans lequel le premier groupement fonctionnel est un alkyne et le deuxième groupement fonctionnel est un azoture.

19. Procédé selon la revendication 14, dans lequel les étapes de a) à f) sont exécutées selon une procédure itérative de préparation d'une nouvelle première bibliothèque de composés et/ou d'une nouvelle deuxième bibliothèque de composés et de nouveau tri jusqu'à ce qu'un candidat-sonde d'imagerie ayant des propriétés optimisées de liaison, de biodistribution, métaboliques et pharmacocinétiques soit identifié.

20. Procédé selon la revendication 14, dans lequel le composé ligand identifié est marqué avec l'isotope radioactif, et le composé ligand ainsi obtenu est utilisé pour des méthodes d'imagerie choisies parmi le groupe comprenant les méthodes d'imagerie PET, SPECT et optique.

21. Procédé selon la revendication 14, dans lequel la liaison des composés candidats aux sites de liaison enzymatique facilite la réaction de chimie clic en l'absence de tout catalyseur externe ajouté.

22. Procédé selon la revendication 14, dans lequel la première bibliothèque de composés candidats et/ou la deuxième bibliothèque de composés candidats comprennent en outre une liaison entre le composé et le premier groupement fonctionnel et/ou une liaison entre le composé et le deuxième groupement fonctionnel.

23. Procédé selon la revendication 22, dans lequel la liaison comprend entre 1 et 10 atomes dans la chaîne de liaison entre le composé et le groupement fonctionnel.

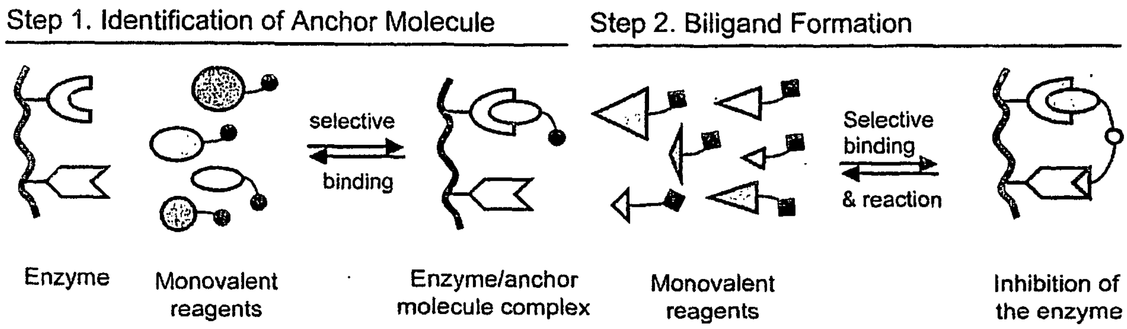
Step 1. Identification of Anchor Molecule
Step 2. Biligand Formation
selective
binding
Selective
binding
& reaction
Enzyme
Monovalent reagents
Enzyme/anchor molecule complex
Monovalent reagents
Inhibition of the enzyme

FIG. 1

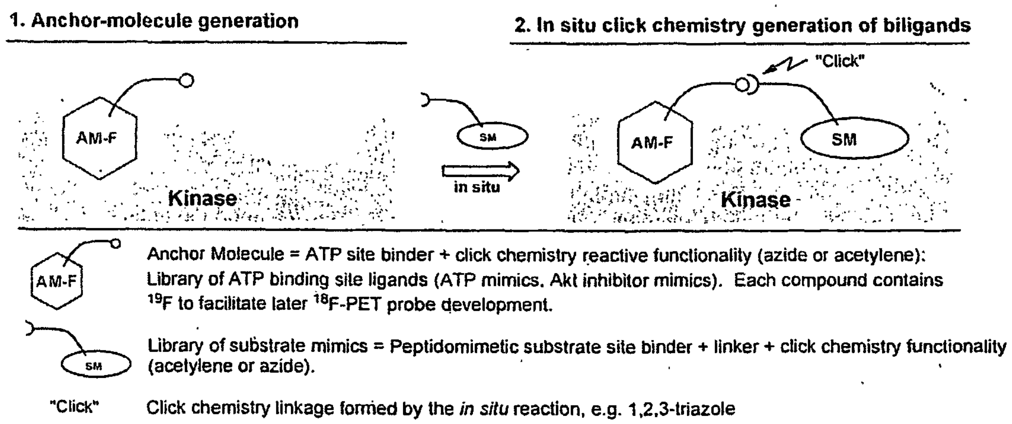
1. Anchor-molecule generation
2. In situ click chemistry generation of biligands
"Click"
AM-F
Kinase
SM
in situ
AM-F
SM
Kinase
AM-F
Anchor Molecule = ATP site binder + click chemistry reactive functionality (azide or acetylene):
Library of ATP binding site ligands (ATP mimics, Akt inhibitor mimics). Each compound contains
$^{19}F$ to facilitate later $^{18}F$-PET probe development.
SM
Library of substrate mimics = Peptidomimetic substrate site binder + linker + click chemistry functionality
(acetylene or azide).
"Click"
Click chemistry linkage formed by the *in situ* reaction, e.g. 1,2,3-triazole

FIG. 2

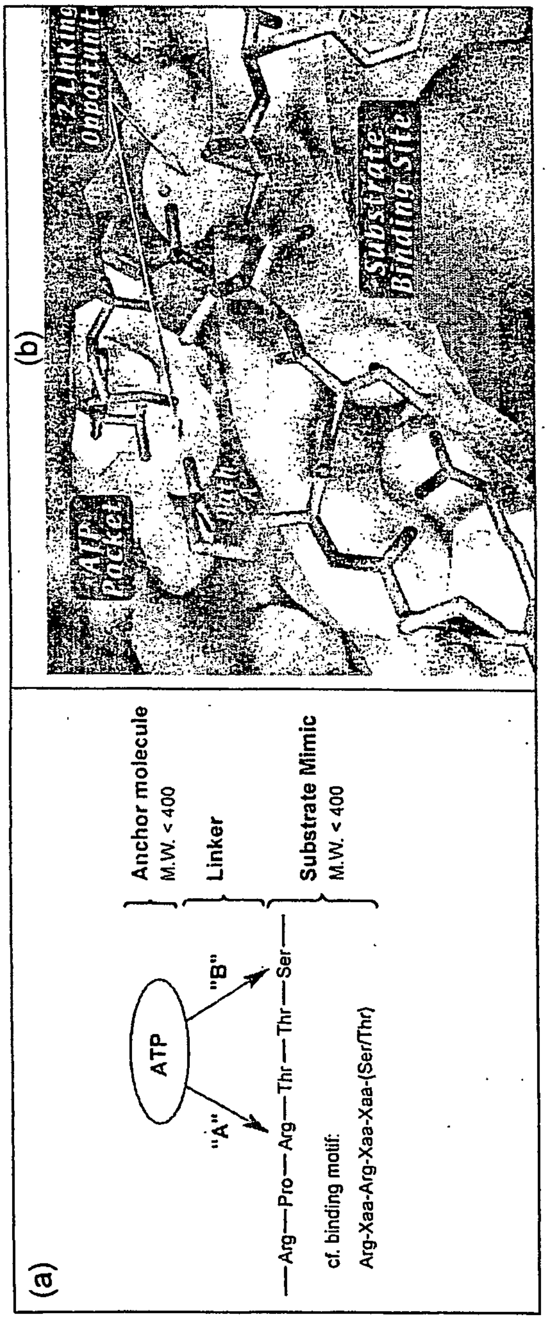
(a)
ATP
"A"
"B"
—Arg—Pro—Arg—Thr—Thr—Ser—
cf. binding motif:
Arg-Xaa-Arg-Xaa-Xaa-(Ser/Thr)
Anchor molecule
M.W. < 400
Linker
Substrate Mimic
M.W. < 400
(b)
ATP Pocket
Substrate Binding Site

FIG. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0167103 A


### Non-patent literature cited in the description


- **Krasinsky et al.** *JACS,* 2005, vol. 127, 6686-6692
- **Manetsch et al.** *JACS,* 2004, vol. 126, 12809-12818
- **Mocharla, V. P. ; Colasson, B. ; Lee, L. V. ; Roeper, S. ; Sharpless, K. B. ; Wong, C.-H. ; Kolb, H. C.** *Angew. Chem. Int. Ed.,* 2005, vol. 44, 116-120
- **Kolb, H. C. ; Finn, M. G. ; Sharpless, K. B.** *Angewandte Chemie, International Edition,* 2001, vol. 40, 2004-2021
- **Kolb, H. C. ; Sharpless, K. B.** *Drug Discovery Today,* 2003, vol. 8, 1128-1137
- **Rostovtsev, V. V. ; Green, L. G. ; Fokin, V. V. ; Sharpless, K. B.** *Angewandte Chemie, International Edition,* 2002, vol. 41, 2596-2599
- **Tornøe, C. W. ; Christensen, C. ; Meldal, M.** *Journal of Organic Chemistry,* 2002, vol. 67, 3057-3064
- **Wang, Q. ; Chan, T. R. ; Hilgraf, R. ; Fokin, V. V. ; Sharpless, K. B. ; Finn, M. G.** *Journal of the American Chemical Society,* 2003, vol. 125, 3192-3193
- **Lee, L. V. ; Mitchell, M. L. ; Huang, S.-J. ; Fokin, V. V. ; Sharpless, K. B. ; Wong, C.-H.** *Journal of the American Chemical Society,* 2003, vol. 125, 9588-9589
- **Lewis, W. G. ; Green, L. G. ; Grynszpan, F. ; Radic, Z. ; Carlier, P. R. ; Taylor, P. ; Finn, M. G. ; Barry, K.** *Angew. Chem., Int. Ed.,* 2002, vol. 41, 1053-1057
- **Manetsch, R. ; Krasinski, A. ; Radic, Z. ; Raushel, J. ; Taylor, P. ; Sharpless, K. B. ; Kolb, H. C.** *Journal of the American Chemical Society,* 2004, vol. 126, 12809-12818
- **M. Whiting ; J. Muldoon ; Y.-C. Lin ; S. M. Silverman ; W. Lindstrom ; A. J. Olson ; H. C. Kolb ; M. G. Finn ; K. B. Sharpless ; J. H. Elder.** *Angew. Chem.,* 2006, vol. 118, 1463-1467
- *Angew. Chem. Int. Ed. Engl.,* 2006, vol. 45, 1435-1439
- **Krasinski, A. ; Fokin, V.V. ; Barry, K.** *Organic Letters,* 2004, 1237-1240
- **Huisgen.** 1,3-Dipolar Cycloaddition Chemistry. Wiley, vol. 1, 1-176
- **Jorgensen.** *Angew. Chem. Int. Ed. Engl.,* 2000, vol. 39, 3558-3588
- **Tietze, L.F. ; Kettschau, G.** *Top. Curr. Chem.,* 1997, vol. 189, 1-120
- **Whitesides et al.** *J. Am. Chem. Soc.,* 1994, vol. 116, 5057
- **J.C. Kemohan.** *J. Biol. Chem.,* 1967, vol. 242, 5813
- *J. Organomet. Chem.,* 1997, vol. 544, 163-174
- *Org. Lett.,* 2005, vol. 7, 577-579
- **Talley, J. J. et al.** *J. Med. Chem.,* 2000, vol. 43, 775-777